Europäisches Patentamt

European Patent Office

Office européen des brevets

(11) Publication number: 0 387 457
A1

(12) EUROPEAN PATENT APPLICATION

(21) Application number: 89400047.0

(22) Date of filing: 06.01.89

(51) Int. Cl.5: C12N 15/16, C12N 15/18, C07K 13/00, C12P 21/02, C12N 1/21, A23K 1/165, //(C12N1/21,C12R1:19)

A request for correction of Figures II, IIbis and III has been filed pursuant to Rule 88 EPC. A decision on the request will be taken during the proceedings before the Examining Division (Guidelines for Examination in the EPO, A-V, 2.2).

The title of the invention has been amended (Guidelines for Examination in the EPO, A-III, 7.3).

(43) Date of publication of application:
19.09.90 Bulletin 90/38

(84) Designated Contracting States:
AT BE CH DE ES FR GB GR IT LI LU NL SE

(71) Applicant: EUROGENTEC S.A.
Campus du Sart-Tilman Allée du Six Août B6 Bldg
B-4000 Liège(BE)

(72) Inventor: Rentier-Delrue, Françoise
Rue de la Magree 25
B-4163 Tauier(BE)
Inventor: Martial, Joseph
Rue Vignoble 13
B-4050 Esneux(BE)
Inventor: Renard, André
Avenue des Cerfs 37
B-4900 Liège(BE)

(74) Representative: Grosset-Fournier, Chantal Catherine et al
SC Ernest Gutmann/Yves Plasseraud 67 boulevard Haussmann
F-75008 Paris(FR)

(54) Recombinant fish hormone proteins.

(57) The present invention relates to pituitary hormone proteins, prolactin and growth hormones from warm water fish, produced by microorganisms harbouring genes cloned from said fish species, which exhibit growth stimulation and osmoregulatory effects, and which display improved resistance to higher temperatures as compared to homologous proteins derived from cold water fish.

The pituitary hormone proteins can be used in the industrial culture of fish to enhance the growth of the fish and to improve the adaptation of the fish to the salinity of the water...

# A PROCESS FOR PRODUCING RECOMBINANT FISH HORMONE PROTEINS EXHIBITING GROWTH PROMOTING AND OSMOREGULATORY EFFECTS, THERAPEUTIC PRODUCTS BASED THEREON AND RECOMBINANT DNA FRAGMENTS CONTAINING DNA SEQUENCES ENCODING SAID FISH GROWTH HORMONES

The present invention relates to a process for producing large quantities of recombinant fish hormone proteins, prolactins and growth hormones exhibiting growth stimulation and osmoregulatory effects, in microorganisms harbouring genes cloned from warm water fish species, coding for said hormone proteins.

Prolactins and growth hormones constitute a family of evolutionary related proteins produced by the pituitary gland in all vertebrates. In general, these pituitary hormone proteins display pleiotrophic biological activities. In fish these activities range from growth stimulation and osmoregulatory adaptation to water salinity, to reproductive metabolism and behavior and mucus production. Whereas the growth hormones (hereafter denoted GH) are involved in growth promotion and osmoregulation, the prolactins (hereafter denoted PRL) exhibit a broad variety of physiological effects on osmoregulation, reproduction, metabolism, behavior and mucus production. Most studies of these hormones have focussed on their growth promoting and osmoregulatory activities, demonstrating their role in freshwater adaptation, (Clarke and Bern, 1980 ; Loretz and Bern 1982 ; Hirano 1986) and suggesting their potential application in the field of fish culture. Recently, several growth hormones and prolactins have been isolated and cloned from various cold water fish species such as salmon, trout, carp and tuna (Sebine et al. 1985 ; Agellon and Chen 1986 ; Nicoll et al. 1988 ; Gonzalez-Villasenor 1988 ; Sato et al. 1988 ; Yasuda et al. 1987 ; Song et al. 1988). Attemps to produce recombinant growth hormones and prolactins by expressing genes cloned from these fish in microorganisms have thus far met with little success.

In the present invention cloning techniques have been used to isolate the genes coding for the different growth promoting and osmoregulatory pituitary hormones of the warm water fish species such as tilapia or catfish. The warm water fish tilapia is a euryhaline fish which can reproduce in sea water as well as in fresh water. Because of recent development of tilapia aquaculture in brackish or in seawater, interest has focussed on growth promotion and salinity tolerance and hence on the physiology of osmoregulation in this species. Earlier studies have shown that prolactin plays an important role in the control of hydromineral balance in tilapia (Dharmamba 1970 ; Dharmamba and Maetz 1972, 1976 ; Clarke 1973 ; Foskett et al. 1982). While little is known about the tilapia GH, the prolactins are well characterized. Two prolactins of different molecular weights have been isolated from the pituitary gland of the tilapia species *Oreochromis mossambicus* (Specker et al. 1985 ; Yamagushi et al. 1988). Amino acid sequence analysis revealed that the two proteins display a high degree of sequence homology (Yamagushi et al. 1988). Studies on the osmoregulatory activities of the two prolactins failed to show any difference between them (Specket et al. 1985 ; Young et al. 1988).

The present invention relates to a process for producing recombinant fish growth hormones and prolactins in microorganisms using the genes encoding for said hormone proteins isolated from warm water fish species particularly tilapia or catfish. The process comprises producing recombinant polypeptides or proteins in typical microorganisms such as Escherichia coli and yeast at a temperature from about 28°C to about 42°C, depending upon the selected microorganism, as a means of obtaining large quantities of stable polypeptides or proteins encoded by cloned genes. By way of example the tilapia species *Oreochromis niloticus* has been used and it is shown that three different hormone products exhibiting growth promoting and/or osmoregulatory activities can be isolated, namely one growth hormone protein (hereafter denoted TGH) and two different prolactins (hereafter denoted respectively TPRLI and TPRLII).

The present invention relates to the culturing of an appropriate host organism previously transformed with a DNA containing nucleotide sequences encoding the tilapia or catfish growth hormones and prolactins which are under the control of regulatory elements such as a promoter recognized by the RNA polymerase of the host organism, a translation initiation and termination signal, and the purification of the protein products synthesized by the host organism.

The invention also relates to new stable proteins or polypeptides.

The invention also relates to the nucleic acids, described hereafter, encoding the tilapia or catfish growth hormones and prolactins and the regulatory signals preceding the coding sequences which allow the coding sequences to be expressed in the host organism including promoter signals which control the transcription of said coding sequences and the translation initiation and termination signals.

The invention also relates to the recombinant DNAs, plasmids, cosmids and phages containing nucleic acid sequences encoding the tilapia or catfish growth hormones and prolactins and the genetic elements essential for their replication.

In a particular application of the invention, the recombinant DNAs, described hereafter, are plasmid expression vectors containing a functional origin of replication, regulatory elements allowing the expression, in the host organism, of the sequences coding for the tilapia or catfish growth hormones and prolactins, a promoter which is active in said host organism, and in particular an inducible promotor.

The invention also relates to the host organisms transformed with the recombinant DNAs, described hereabove, capable of replicating in said host organism, and containing regulatory elements allowing the expression of the tilapia or catfish growth hormones and prolactins in this host organism.

A preferred host organism is *Escherichia coli*, transformed with recombinant DNAs as described in the examples below.

The invention also relates in particular to the cultures of host organisms transformed as described below and which are capable of producing the tilapia or catfish growth hormones and prolactins according to the invention.

The present invention also relates to the products based on the tilapia growth hormone proteins and prolactins in the industrial culture of fish to enhance the growth of the fish in culture and to improve their adaptation to culturing conditions.

The present invention also relates to preparations based on tilapia growth hormone proteins and prolactins or mixtures thereof which can be administered to fish cultures using methods such as injection, balneation or spraying.

The present invention also relates to food for fish, containing, besides the classical ingredients, the recombinant polypeptides or proteins produced according to the process of the invention, in an efficient amount.

The process for preparing a stable recombinant polypeptide presenting growth and/or osmoregulatory properties comprises the following steps :
- a cellular host, which is previously transformed by an appropriate vector containing a nucleic acid coding for polypeptides having
. the polypeptide sequence of the natural growth promoting and/or osmoregulatory hormones of tilapia or catfish or
. a polypeptide sequence containing part of the totality of the sequence of the natural growth promoting and/or osmoregulatory hormones of tilapia or catfish, in so far as the polypeptide sequence produced has growth promoting and/or osmoregulatory properties,
is cultivated in an appropriate medium at a temperature from about 28°C to about 42°C depending on the selected microorganism and
- the polypeptide produced by said transformed cellular host is recovered from the above said medium culture and, if need be, purified.

The expression "stable polypeptide" means that the polypeptide obtained is stable, even after isolation from the medium culture in which it is produced. More precisely, it means that the polypeptide is stable at the temperatures of 28°C to 42°C under which it is synthesized in the cellular host, and is not subject to any rapid degradation, and hence can be expressed in large amounts comprising a substantial fraction of the total cellular protein of said cellular host, substantial fraction meaning for example at least 10% to 80% of the total cellular protein in bacterial hosts, or for example at least 3% to 50% of the total cellular protein in yeast cellular hosts.

The expression "recombinant polypeptide" is not limited to mere aminoacid sequences, but also encompasses the glycosylated polypeptides or the polypeptides comprising disulfide bridges. The glycosylated polypeptides can be obtained directly by expressing a nucleic acid above and hereafter defined in an appropriate cellular host, for example yeast, or can be obtained by in vitro glycosylation of a non glycosylated recombinant polypeptide.

The polypeptides comprising the disulfide bridges can be obtained either directly by expressing a nucleic acid, defined above and hereafter, in an appropriate cellular host, for example yeast, or indirectly by denaturation and renaturation of purified fractions of the recombinant polypeptide in buffers containing an appropriate standard redox potential.

The temperature used is chosen such as it is compatible with the conditions required to allow the synthesis of proteins in the cellular host. The upper limit of the temperature range is critical because above that temperature all macromolecular synthesis in the cellular host ceases. The lower limit of the temperature range is critical because under that temperature all enzymatic reactions, including macromolecular synthesis, and protein synthesis, are slowed down to such an extent that the polypeptide will not be produced in the large amounts defined hereabove.

In an advantageous process of the invention, the nucleic acids coding for the natural polypeptides presenting growth promoting and/or osmoregulatory properties are under the control of regulatory elements, such as a promotor recognized by the RNA polymerase of the host organism, a translation initiation and termination signal.

In the process of the invention, the cellular host can advantageously be E. coli, the growth of which is stopped at the exponential phase and the polypeptide produced by said transformed cellular host is recovered from the culture of cells from other proteins produced by E. coli.

E. coli is advantageously used for the production of very large amounts, as the experimental conditions required are well defined.

The cellular host can also be yeast, such as Sacharomycces strains. Yeast is advantageously used for the production of the polypeptide in the culture medium as the polypeptide will be secreted in the culture medium when the gene encoding the polypeptide contains a signal peptide.

Furthermore, yeast is also advantageously used for the production of the biologically active polypeptide since in his host naturally occurring disulfide bridges are formed in newly synthesized proteins.

In the process of the invention, the recombinant polypeptide can be preceded by a signal peptide, particularly by a signal peptide having the same amino acid sequence as the natural signal peptide in tilapia or catfish.

In the process of the invention, the recombinant polypeptide is advantageously encoded by a gene in which the coding sequence of the polypeptides is preceded by an initiator methionine codon.

The latter is required for allowing the translation of the polypeptide to be initiated at the correct codon. The preceding methionine might or not be cleaved off from the polypeptide during its synthesis.

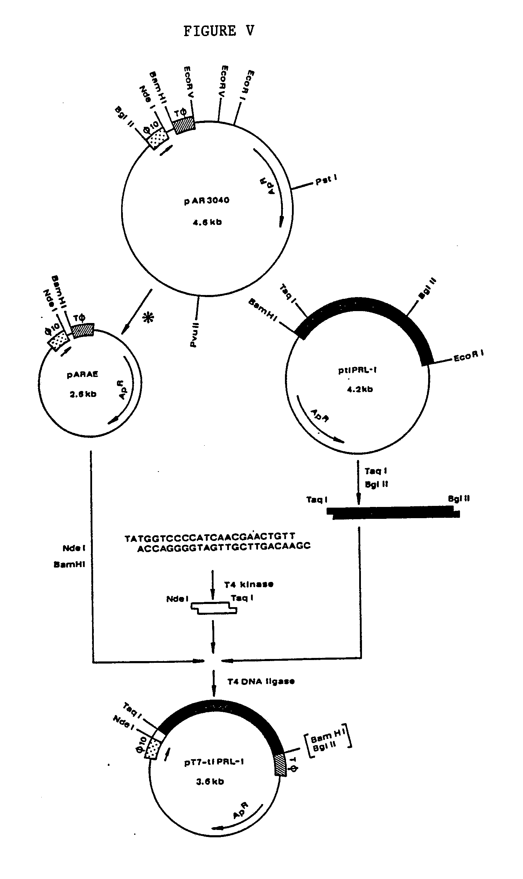

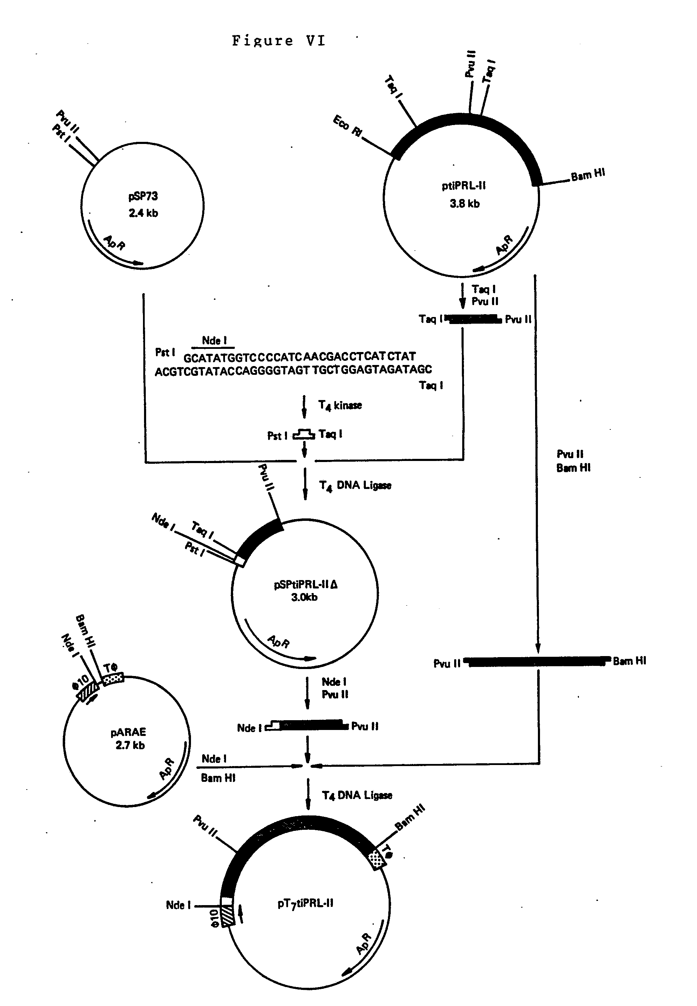

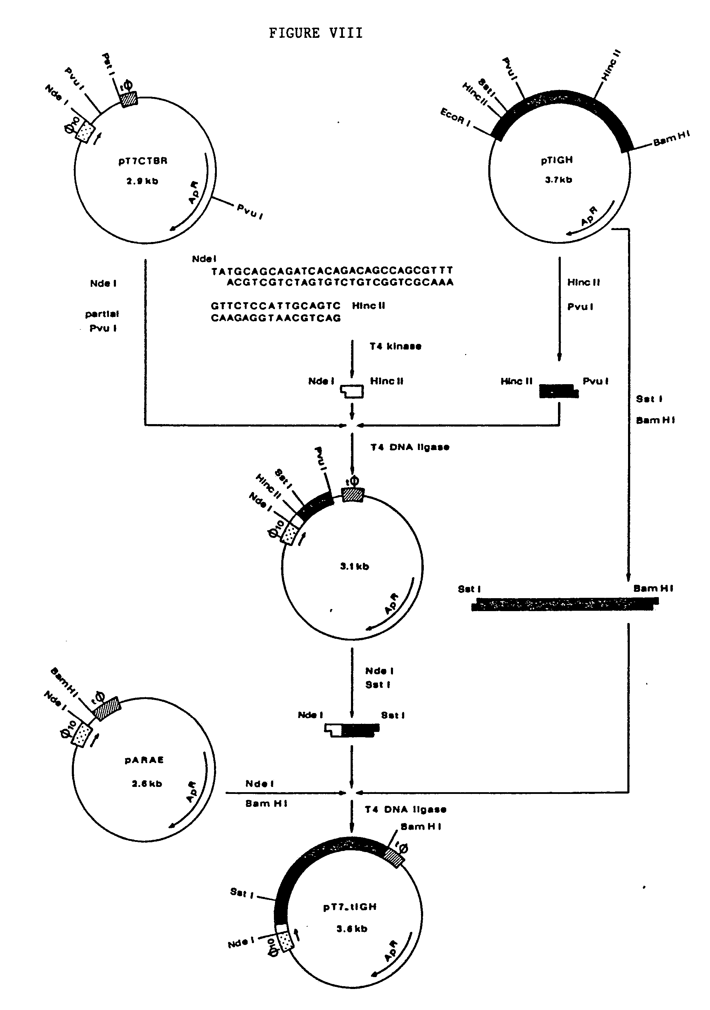

The process of the invention enables particularly to prepare the following recombinant polypeptides : those comprising in their polypeptide chain an aminoacid sequence contained in one of the following amino sequences :
* from amino acid (-24) to amino acid (188) represented in Fig. 2,
* from amino acid (-24) to amino acid (188) represented in fig. 2bis,
* from amino acid (-23) to amino acid (177) represented in Fig. 3,
* from amino acid (-20) to amino acid (187) represented in Fig. 7,
* from amino acid (1) to amino acid (188) represented in Fig. 2,
* from amino acid (1) to amino acid (188) represented in Fig. 2, preceded by Met,
* from amino acid (1) to amino acid (188) represented in fig. 2bis,
* from amino acid (1) to amino acid (188) represented in fig. 2bis, preceded by Met,
* from amino acid (1) to amino acid (177) represented in Fig. 3,
* from amino acid (1) to amino acid (177) represented in Fig 3, preceded by Met,
* from amino acid (1) to amino acid (187) represented in Fig. 7,
* from amino acid (1) to amino acid (187) represented in Fig. 7, preceded by Met,
or those comprising one of the above amino acid sequences,
or those constituted by one of the above mentioned amino acid sequences.

The process of the invention also enables to prepare the recombinant polypeptides comprising in their polypeptide chain an amino acid sequence contained in one of the following sequences :
* from amino acid (1) to amino acid (188) represented in fig. 2bis,
* from amino acid (1) to amino acid (177) represented in Fig. 3,
or those comprising one of the above amino acid sequences
or those constituted by one of the above mentioned amino acid sequences.

More particularly, the process of the invention enables to prepare new polypeptides
- comprising in their polypeptide chain an amino acid sequence contained in one of the following sequences:
* from amino acid (-20) to amino acid (187) represented in Fig. 7,
* from amino acid (1) to amino acid (187) represented in Fig. 7,
* from amino acid (1) to amino acid (187) represented in Fig. 7, preceded by Met,
- or comprising one of the abovesaid amino acid sequences or constituted by one of the abovesaid amino acid sequences,
- or comprising or being constituted by one of the following amino acid sequences :
* from amino acid (-24) to amino acid (188) represented in Fig. 2,
* from amino acid (-24) to amino acid (188) represented in Fig. 2 bis,
* from amino acid (-23) to amino acid (177) represented in fig. 3,
* from amino acid (1) to amino acid (188) represented in fig. 2,
* from amino acid (1) to amino acid (188) represented in Fig. 2, preceded by Met,
* from amino acid (1) to amino acid (188) represented in Fig. 2 bis, preceded by Met,
* from amino acid (1) to amino acid (177) represented in Fig. 3, preceded by Met.

These new polypeptides are also part of the invention.

The polypeptides of the invention can not be prepared only by the process of the invention, but can also be prepared by any preparation process, such as classical chemical synthesis.

The invention also relates to the signal peptides constituted by the following aminoacid sequences:

- from aminoacid (-24) to aminoacid (-1) of fig. 2,
- from aminoacid (-23) to aminoacid (-1) of fig.3,
- from aminoacid (-20) to aminoacid (-1) of fig.7.

The invention relates to any recombinant polypeptide constituted by anyone of the above mentioned signal peptides, combined with any other hormones such as other prolactins or growth hormones of fish.

The invention also relates to the nucleic acids comprising a nucleotide chain coding for the following aminoacid sequence; - those contained in one of the following sequences :

* from amino acid (-20) to amino acid (187) represented in Fig. 7,
* from amino acid (1) to amino acid (187) represented in Fig. 7,
* from amino acid (1) to amino acid (187) represented in Fig. 7, preceded by Met,
- or those comprising one of the abovesaid amino acid sequence or constituted by one of the abovesaid amino acid sequences,
- or comprising or being constituted by one of the following amino acid sequences :
* from amino acid (-24) to amino acid (188) represented in Fig. 2,
* from amino acid (-24) to amino acid (188) represented in Fig. 2 bis,
* from amino acid (-23) to amino acid (177) represented in fig. 3,
* from amino acid (1) to amino acid (188) represented in fig. 2,
* from amino acid (1) to amino acid (188) represented in Fig. 2, preceded by Met,
* from amino acid (1) to amino acid (188) represented in Fig. 2 bis, preceded by Met,
* from amino acid (1) to amino acid (177) represented in Fig. 3, preceded by Met.

Advantageous nucleic acids of the invention can also be defined as those comprising a part or the totality of one of the following nucleotidic sequences :

* from nucleotide (-26) to nucleotide (Z) of Fig. 7,
* from nucleotide (1) to nucleotide (621) of figure 7,
* from nucleotide (61) to nucleotide (621) of Fig. 7,
* from nucleotide (61) to nucleotide (621) of Fig. 7, preceded by the AUG codon,
or constituted by one of the above mentioned nucleotide sequences.

Other nucleic acids of the invention are those comprising one of the following nucleotidic sequences :

* from nucleotide (-50) to nucleotide (x) of fig.2,
* from nucleotide (1) to nucleotide (636) of fig.2,
* from nucleotide (73) to nucleotide (636) of fig. 2,
* from nucleotide (73) to nucleotide (636) of fig. 2, preceded by the AUG codon,
* from nucleotide (-38) to nucleotide (y) of fig. 3,
* from nucleotide (1) to nucleotide (600) of fig. 3,

* from nucleotide (70) to nucleotide (600) of fig. 3,
* from nucleotide (70) to nucleotide (600) of fig.3, preceded by the AUG codon,
* from nucleotide (-50) to nucleotide (x) of Fig. 2bis,
* from nucleotide (1) to nucleotide (636) of Fig. 2 bis,
* from nucleotide (73) to nucleotide (636) of Fig. 2 bis,
* from nucleotide (73) to nucleotide (636) of Fig. 2 bis, preceded by the AUG codon,
or constituted by one of the above mentioned nucleotidic sequences.

The invention also relates to the nucleic acids coding for the above defined signal peptides.

The invention also relates to recombinant vectors, particularly for the cloning and/or expression, particularly of the plasmid, cosmid or phage type, containing a nucleic acid coding for a natural polypeptide - presenting growth promoting and/or osmoregulatory properties - of tilapia or catfish in particular in one of their sites which is not essential for their replication, and in particular containing the nucleic acids coding for one of the following polypeptides :

* from amino acid (-24) to amino acid (188) represented in Fig. 2,
* from amino acid (-24) to amino acid (188) represented in fig. 2bis,
*from amino acid (-23) to amino acid (177) represented in Fig. 3,
* from amino acid (-20) to amino acid (187) represented in Fig. 7,
* from amino acid (1) to amino acid (188) represented in Fig. 2,
* from amino acid (1) to amino acid (188) represented in Fig. 2, preceded by Met,
* from amino acid (1) to amino acid (188) represented in fig. 2bis,
* from amino acid (1) to amino acid (188) represented in fig. 2bis, preceded by Met,
* from amino acid (1) to amino acid (177) represented in Fig. 3,
* from amino acid (1) to amino acid (177) represented in Fig. 3, preceded by Met,
* from amino acid (1) to amino acid (187) represented in Fig. 7,
* from amino acid (1) to amino acid (187) represented in Fig. 7, preceded by Met.

Advantageous recombinant vectors of the invention contains :
- regulatory elements necessary to allow the expression of a sequence of amino acids of a polypeptide presenting growth promoting and/or osmoregulatory properties of tilapia or catfish in a cellular host and
- possibly a promoter, particularly an inducible promoter.

The invention also relates to cellular hosts which are transformed by recombinant vectors of the invention and comprising the regulation elements enabling the expression of the nucleic sequence coding for the polypeptide presenting growth promoting and/or osmoregulatory properties of tilapia or catfish in this host.

An advantageous cellular host is E. coli transformed by anyone of the vectors of the invention.

The invention also relates to products for fish containing at least one of the polypeptides such as the ones obtained by the process of the invention, or mixtures thereof and for instance any of the polypeptides of fig. 7 combined with anyone of the polypeptides of fig. 2, or fig. 2 bis or fig. 3, in an efficient amount such as a 5 ng to 1µg of polypeptide/g of fish per day, when administered by injection.

The invention also relates to food for fish containing, besides the classical nutrient ingredients at least anyone of the polypeptides such the ones obtained by the process of the invention, in an efficient amount such as 1µg to 1 mg/ of polypeptide/g of fish food per day.

## RESULTS

In the following disclosure three preferred protein products derived from the tilapia species Oreochromis niloticus will be described, by way of examplification only, namely one growth hormone (hereinafter referred to as TGH) and two prolactins of different molecular size (hereinafter referred to as TPRLI and TPRLII respectively). The prolactins and growth hormone products were obtained by isolating the genes coding for these hormones from a c-DNA library and expressing the cloned genes in strains of *Escherichia coli*, according to the procedures described hereafter.

### cDNA library construction

In order to facilitate the isolation of the hormone genes, a c-DNA library was constructed using m-RNA preparations isolated from the anterior part of the pituitary gland where these hormones are synthesized in large quantities. First was prepared total RNA from 1 gram of tissue using the guanidium isothiocyanate procedure (Ulrich et al., 1977; Chirgwin et al., 1979), whereafter the poly A$^+$ m-RNA fraction was purified by chromatography on oligo (dT) cellulose. The poly A$^+$ m-RNA was then used to synthesize double stranded cDNA according the procedure described by Gubler and Hoffman (1983). The double stranded cDNA was size fractionated by chromatography on sepharose 4B (Pharmacia) and the fractions containing DNA fragments larger than 700 base pairs were pooled. The blunt-ended c-DNA molecules were inserted into the pUC13 plasmid (Vieria and Messing, 1982) previously digested with SmaI and dephosphorylated. The ligated products were then transformed into *E. coli* RR1ΔM15 (lac Z, M15, F$^r$, laq I$^Q$, ZM15, proA) according to the procedure of Mandel and Higa (1970), and clones containing recombinant plasmids were selected on Luria-broth plates containing 100 g/ml ampicillin, 0.5 mM isopropyl-β-D-thiogalactoside (IPTG) and 0,01 % 5-bromo-4-chloro-3-indolyl-β-D-galactopyranoside (X-gal). On these plates, transformants containing the religated pUC13 vector will yield blue colonies whereas the transformants containing recombinant pUC13 plasmids will give rise to white colonies. Starting from 2 g of polyA$^+$ m-RNA, we obtained a c-DNA library of approximatively 4.200 recombinant transformants.

### Screening of the clones

2000 White colonies of the c-DNA library were transferred to nitrocellulose filters, grown overnight, lysed, neutralized and fixed as described in Maniatis (1980). The filters were then hybridized to appropriate $^{32}$p-labelled probes described below. Prehybridization and hybridization were carried out in 6X SSC- 1X Denhardt's solution containing boiled, sheared salmon sperm DNA for 16 hours at a temperature of 45 C (1X SSC : 0.15 M Nacl / 0.015M Na citrate; 1X Denhardt's : 0,02% of each ficoll, polyvinylpyrolidone and bovine serum albumin). The filters were first washed in 6X SCC / 1X Denhardt's for 30 minutes at room temperature and then washed for 30 minutes at temperatures of 54 C.

### Example 1 : Prolactin products

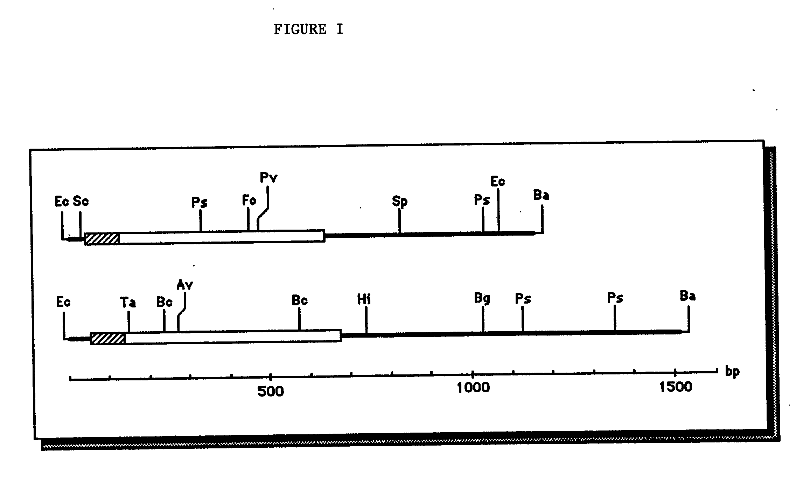

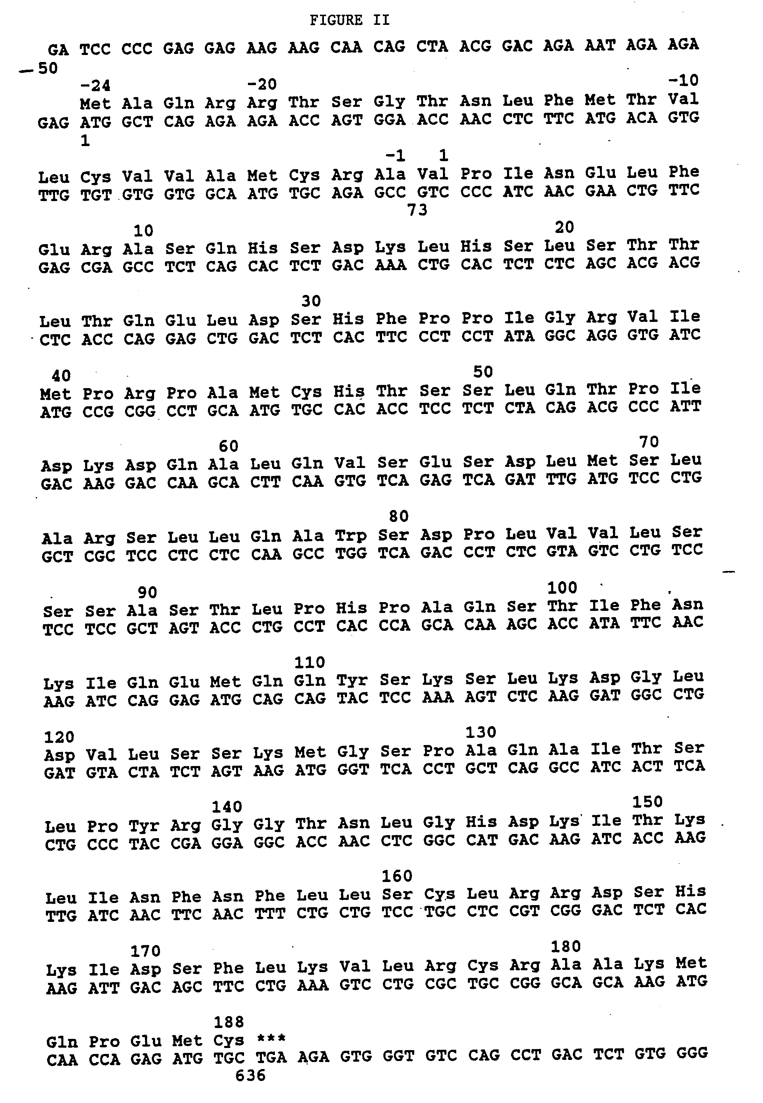

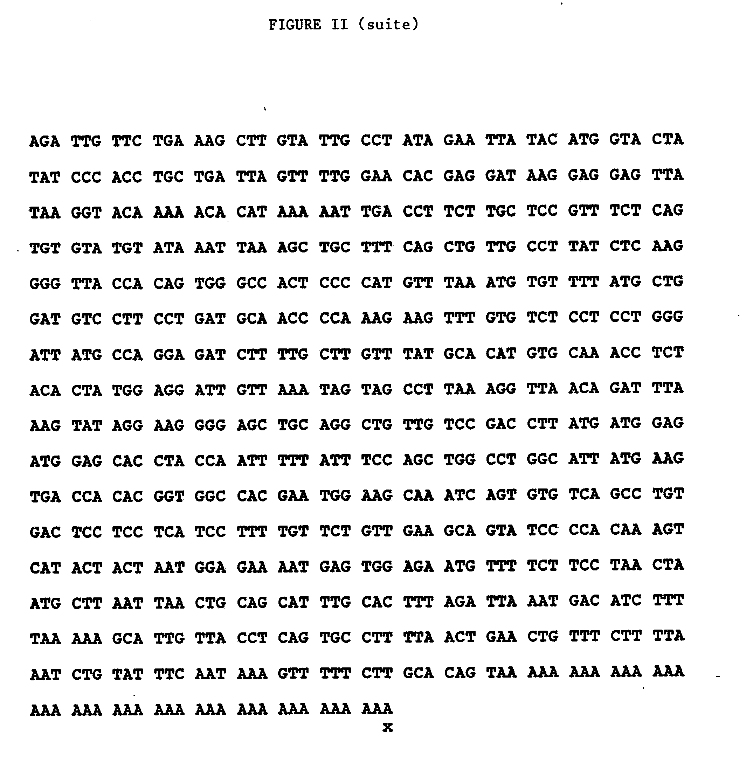

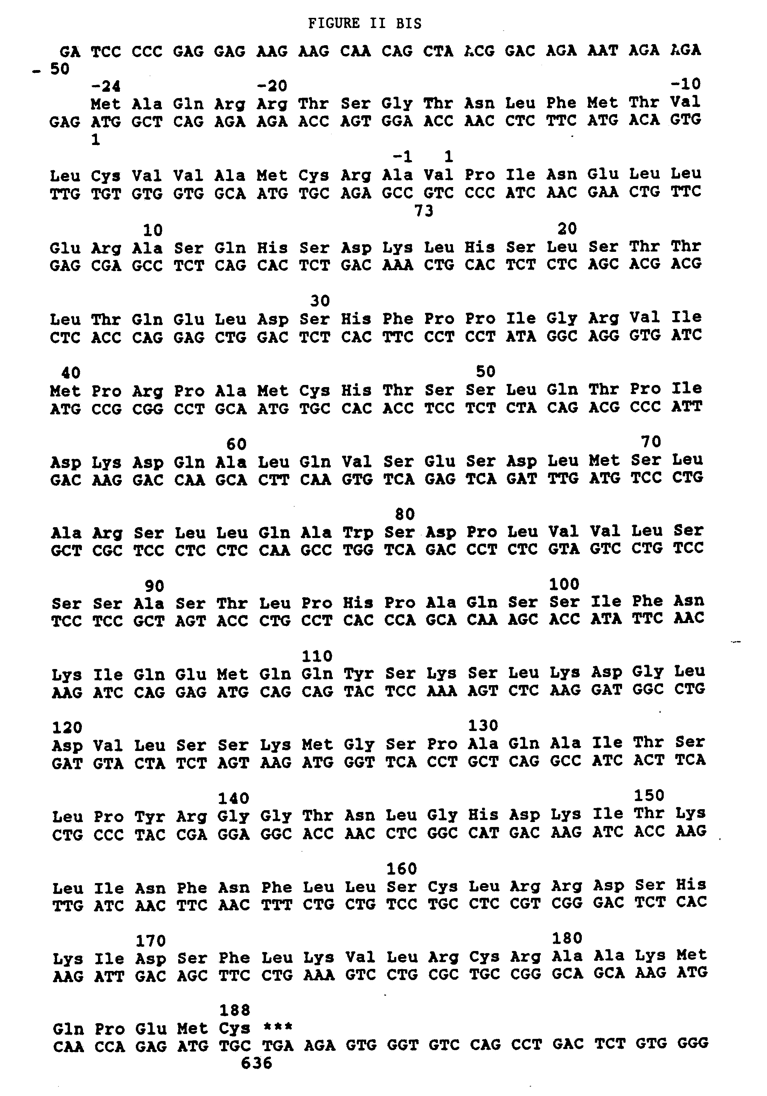

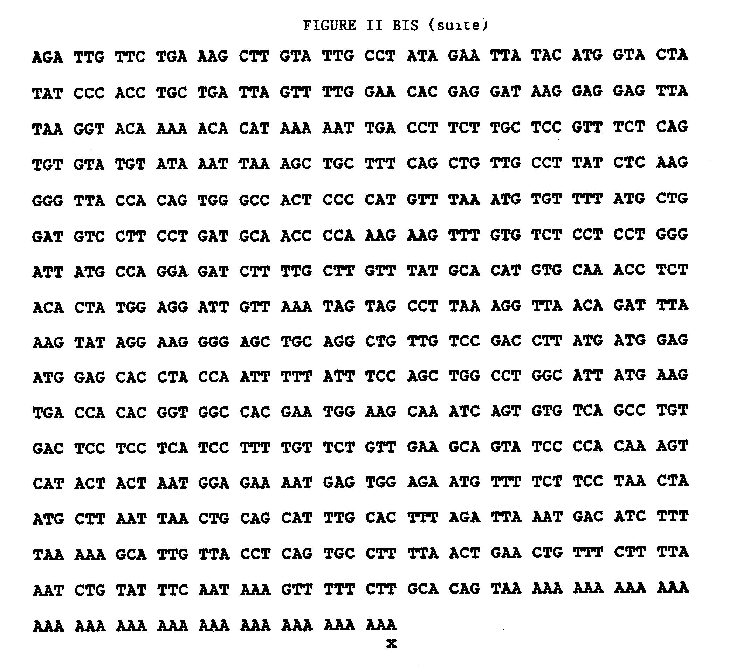

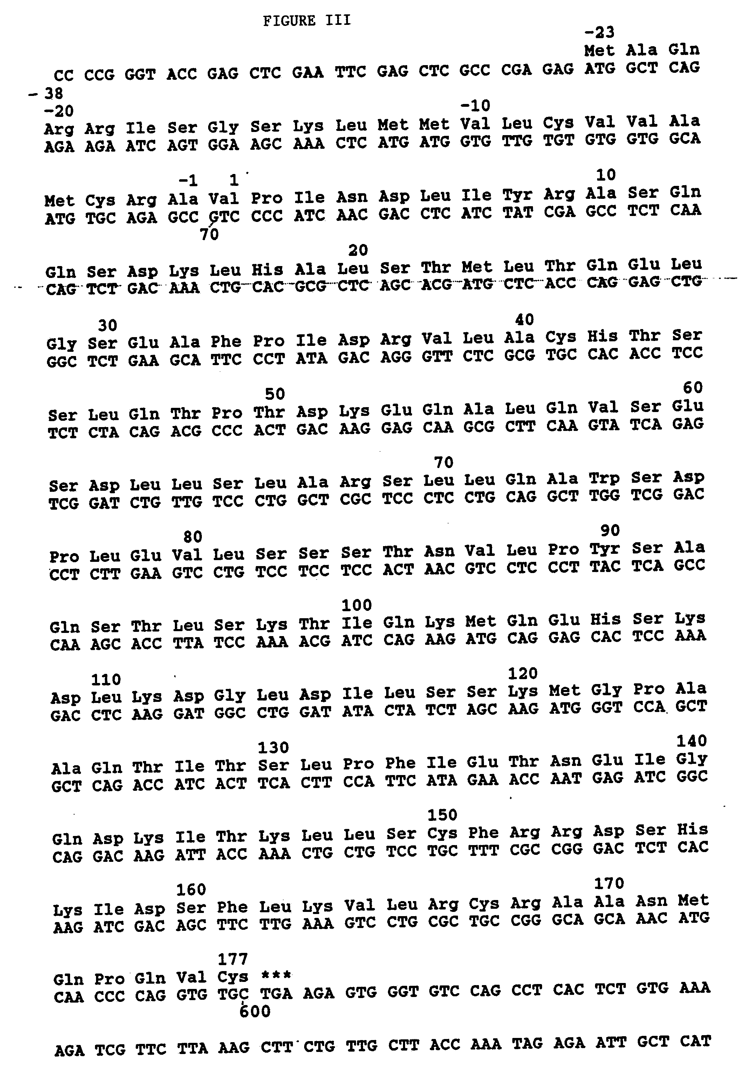

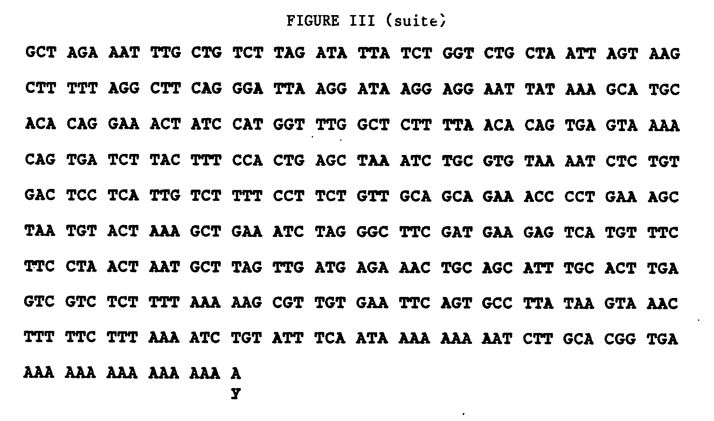

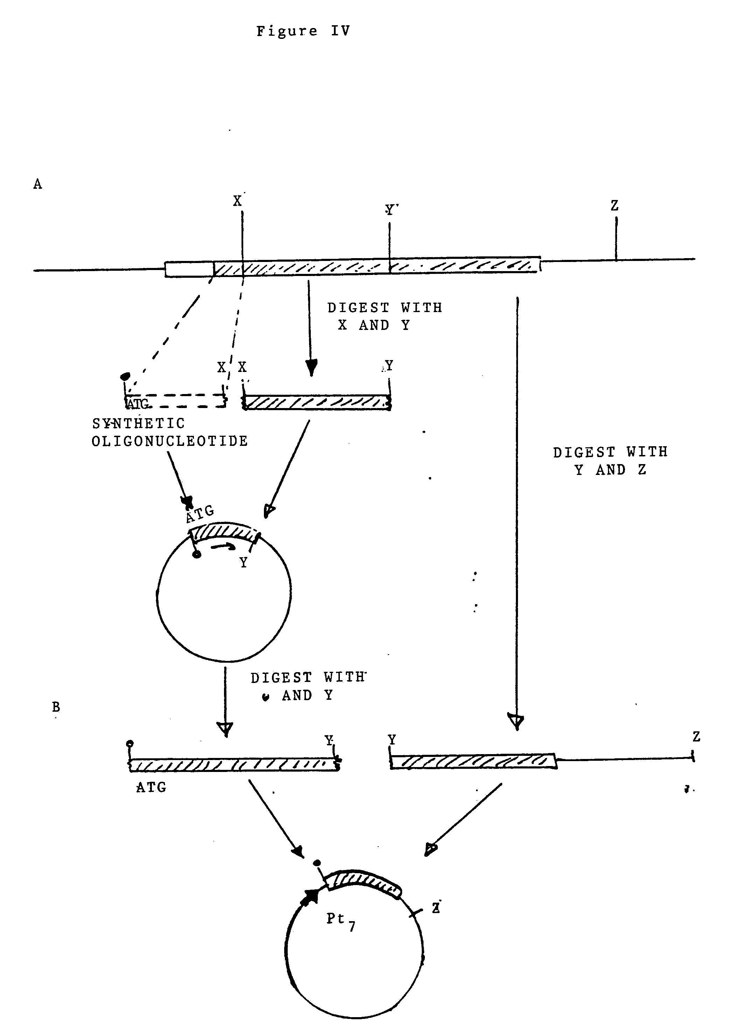

The cDNA clones coding for the tilapia prolactins were isolated by screening the cDNA library with a $^{32}$P labelled 700bb fragment of the trout prolactin gene corresponding to the entire coding region of the gene (Mercier et al., 1989). After hybridization, 156 clones out of 2000 clones were found to hybridize selectively. The recombinant plasmids of 50 of these clones were extracted, sized by electrophosesis on a 1 % agarose gel and analyzed by restriction enzyme cleavage. We identified 22 full-length clones containing long cDNA inserts which could be grouped into two types based on their restriction maps. Figure 1 shows the restriction maps of both types of cDNA designated TPRLI and TPRLII. TPRLI has a length of 1473 basepairs and contains one AvaII, BgIII, HindIII, TaqI site and two BcII and PstI sites PRLII has a length of 1150 bp and contains one EcoRI, FokI, PvuII, SacI and SphI site, two PstI sites and three TaqI sites. Sixteen recombinant plasmids out of 22 were of type I while 8 were of type II. Two representative plasmids of each type were chosen for further study and were designated pTPRLI and pTPRLII respectively.

Characterization of the TPRLI and TPRLII genes

In order to confirm that the TPRLI and TPRLII c-DNA encode tilapia prolactin proteins, we determined the nucleotide sequences of the two cDNA fragments after recloning them into the single stranded phage vectors M13mp18 and M13mp19 vectors (Yanish-Perron et al., 1982). The complete nucleotide sequences of the TPRLI and the TPRLII cDNA inserts are shown in figures 2 and 3.

The TPRLI cDNA contains a large open reading frame 636 nucleotides long followed by a 3′ untranslated region of 787 bp which contains the hexanucleotide AATAAA which precedes the polyadenylation signal of eukaryotic mRNAs. The polypeptide encoded by the open reading frame is 212 amino acids long. Comparison of the amino acid sequence of this polypeptide deduced from the nucleotide sequence (figure 2) with the sequence of the PRL188 protein isolated from the tilapia sepcies *Oreochromis mossambicus* - (Yamaguchi et al., 1988), reveals that, except for the first 25 amino acids at the NH₂ terminus, which are not found in the PRL188 protein, both sequences are almost identical except for two amino acid substitutions at portions 7 and 100. The absence of this polypeptide in the mature protein and the fact that it has a high incidence of hydrophobic amino acid residues, indicate that this segment may act as a signal peptide which is cleaved off after secretion. We conclude that the mature prolactin encoded by the TPRLI cDNA has a length of 188 amino acids with a molecular weight of 20,891 Da and starts at valine residue number 1 (figure 2).

The nucleotide sequence of the TPRLII cDNA contains a large open reading frame of 600 bp followed by a 3′ untranslated region of 512 bp containing a polyadenylation signal. The coding region of TPRLII encodes a polypeptide of 200 amino acids. Comparison of the amino acid sequence of this polypeptide deduced from the nucleotide sequence (figure 3) with the amino acid sequence of the TPRL177 protein isolated form the *Oreochromis mossambicus* species (Yamaguchi et al., 1988), reveals that both sequences are identical, except for the first 23 amino acids encoded by the TPRLII cDNA which are not found in the TPRL177 protein. Like in the case of the TPRLI gene this segment of 23 amino acids corresponds presumably to the signal peptide which is cleaved off after secretion. One concludes that the mature TPRLII protein is 177 amino acids long with a molecular weight of 19,591 Da and starts at valine residue number 1 (figure 3). The TPRLI cDNA is 311 nucleotides longer than TPRLII cDNA, due to a longer coding region (36 nucleotides and a longer 3′ untranslated region (270 nucleotides). Comparison of amino acid sequences deduced from the nucleotide sequences shows that the two proteins are very homologous : TPRLII protein is 11 amino acids shorter than TPRLI protein, and the two proteins differ by 50 amino acid substitutions which are randomly distributed.

Expression of the TPRL genes

The strategy used to express the TPRL genes in E. coli was essentially as described by Studier and Moffat, 1986. In brief, the TPRL genes are inserted behind the strong promoter of gene 10 of bacteriophage T7 in such a way that the translation of the inserted genes starts at the ribosome binding site of gene 10. Furthermore, we modified the beginning of the TPRL genes so as to remove the signal peptide coding region, in such a way that the initiator ATG is followed immediately by the first codon of the mature TPRL proteins. In this way, the bacteria will synthesize directly the mature TPRL proteins without their respective signal peptides.

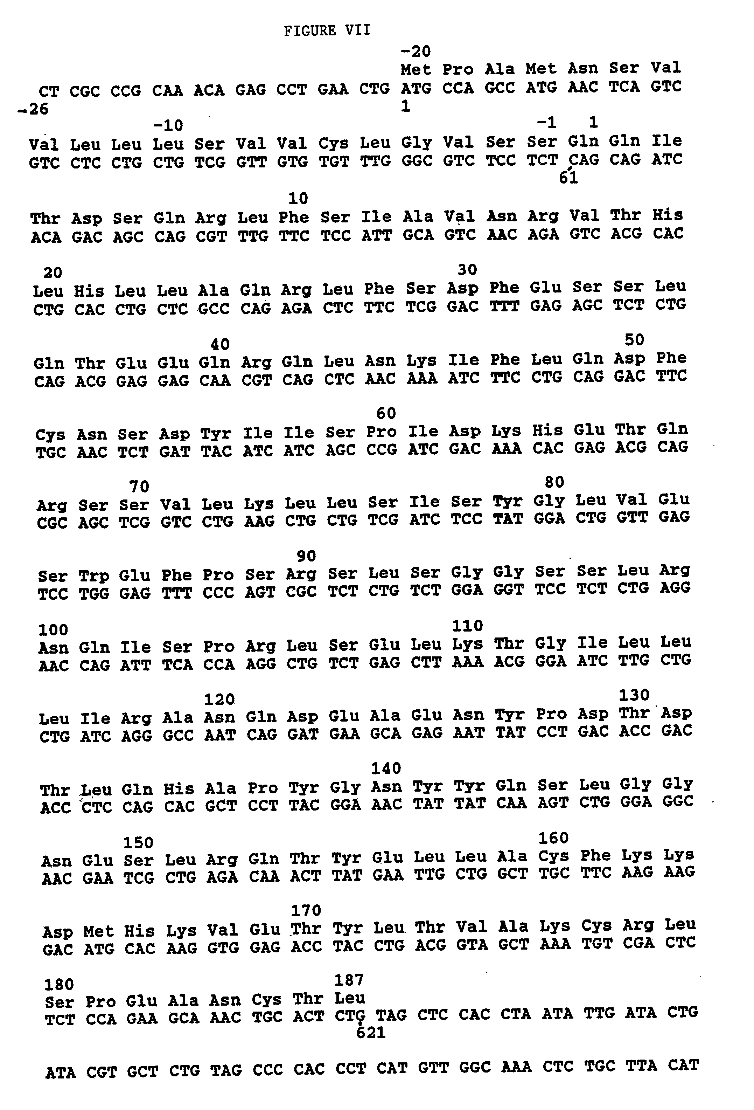

The general scheme for constructing the plasmids expressing the TPRL genes, illustrated in figure 4, involves two steps : in a first step we modified the 5′ ends of the TPRL genes by combining an internal restriction enzyme fragment of the gene with a synthetic oligonucleotide containing an initiator AGT followed by the missing codons of the gene up to the restriction fragment. In a second step we inserted the modified 5′ ends of the genes together with the remaining parts of the genes behind the bacteriophage T7 promoter of the expression vector pARAE. The pARAE plasmid is derived from the plasmid vector pAR3040 (Rosenberg et al., 1987) which contains bacteriophage T7 promoter of gene 10 and its natural terminator for T7 RNA polymerase. In the pARAE derivative we have removed the PvuII-BglII and the EcoRI-EcoRV fragments and deleted the PstI site in the beta-lactamase gene (figure 5).

The construction of the plasmid pTPRLI which expresses the TPRLI protein is diagrammed in figure 5. In this case the engineering of the modified TPRLI gene behind the bacteriophage T7 promoter of plasmid pARAE was carried out in a single step by ligating 3 fragments : (a) the pARAE plasmid cleaved with NdeI and BamHI, (b) a TaqI - BglII fragment from plasmid pTPRLI which carries almost the entire coding region of the mature TPRLI gene except for the first 7 amino acids and (c) a synthetic double stranded oligonucleotide, shown in figure 5, containing an initiator ATG and the first 7 amino acids of the tiPRL gene and with cohesive ends for NdeI and TaqI. The ligation mixture was used to transform *E. coli* HB101 and transformants containing the desired recombinant plasmid were identified using standard plasmid analysis techniques.

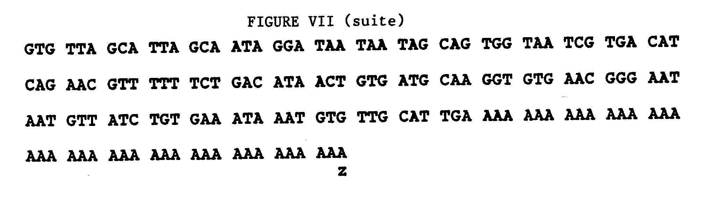

The construction of the plasmid pT7TPRLII diagrammed in figure 6 was performed as follows. In the first step we constructed the plasmit pTPRLM containing the modified 5′ end of the TPRLII gene by inserting the synthetic oligonucleotide shown in figure 6 together with a TaqI-PvuII fragment into the plasmid pSP73. The TaqI-PvuII fragment isolated from plasmid ptiPRLII contains the 5′ end of the TPRLII gene except for the first 8 codons for the mature TPRLII protein. The synthetic oligonucleotide contains an initiator ATG comprised within an Nde 1 site, followed by the first 8 codons for the mature protein. pSP73 was cleaved with PstI and PvuII and ligated together with the synthetic oligonucleotide and the TaqI-PvuII fragment purified from plasmid pTGH by electrophoresis on 1 % agarose. The ligation mixture was transformed into *E. coli* HB101 and transformants containing the desired recombinant plasmid pTPRLM were identified using standard plasmid analysis techniques. In the second step we reconstructed the TPRLII gene by ligating 2 fragments of the TPRLII gene, the NdeI-PvuII fragment from the plasmid pTPRLM and the PvuII-BamHI fragment from the plasmid pTPRLII, into the expression vector pARAE. Both fragments were purified by agarose gel electrophoresis after digesting both plasmids with the respective restriction enzymes and were ligated together with the plasmid pARAE digested with the enzymes NdeI and BamHI. The ligation mixture was used to transform *E. coli* HB101 and transformants containing the desired recombinant plasmid pT7TPRLII were identified using standard plasmid analysis techniques.

In order to express the TPRL proteins, the pT7TPRLI and pT7TPRLII plasmids were transformed into the *E. coli* strain BL21 (DE3) which produces the bacteriophage T7 RNA polymerase under the control of the lac UV5 promoter (Studier and Moffat, 1986). In the presence of the inducer of the lac promoter, IPTG, the synthesis of the T7 RNA polymerase is switched on, which in turn allows the expression of the genes which are under the control of a T7 promoter. *E. coli* BL21 (DE3) containing the pT7TRL-I plasmid were grown overnight in 5ml Luria-broth in the presence of 100 ug/ml ampicillin. Of this culture, 0.4 ml were used to inoculate 10 ml of the same medium supplemented with ampicillin (100 ug/ml). and 0.4 mM IPTG was added when the culture reached $A_{600}$m = 0.8. After three hours, the cells were harvested and lysed in SDS sample buffer (Laemmli, 1970) and analyzed by sodium dodecylsulfate polyacrylamide gel electrophoresis (SDS-PAGE) with Coomassie blue staining. Comparison of the total cell proteins in extracts of non-induced cultures and IPTG induced cultures shows that in both induced cultures a new abundant protein is produced : a 24 kd protein in the strain harbouring the pT7TPRLI plasmid and a 20 kd in the strain harbouring the pT7TPRLII plasmid. The molecular weights of the newly synthesized proteins correspond to the expected sizes of the two prolactin proteins.

Both newly synthesized proteins are produced in large amounts, reaching over 45 % of the total cellular protein. Western blot analysis of the SDS PAGE using a salmon growth hormone antiserum shows that both newly synthesized proteins cross react with the antiserum, thus demonstrating that the TPRLI and TPRLII proteins correspond to the tilapia prolactin.

The overproduced TPRL proteins are found to be contained within inclusion bodies, which are readily purified after lysing the cells by sonication and centrifugation. The prolactin proteins can be easily purified by solubilizing the purified inclusion bodies in the presence of 8 M urea, whereafter the solution is dialyzed against buffers containing protease inhibitors. The proteins were further purified by chromatography on Sephadex G100.

Example 2 :Growth hormone product

The c-DNA clones coding for the tilapia growth hormone (TGH) were isolated by screening the c-DNA library with a $^{32}$P labelled probe isolated from the plasmid -containing the trout GH gene, as described earlier. As probe was used a FokI-BglII of 394 bp corresponding to the 5′ end of the trout GH gene. The screening of 2000 recombinant white colonies of the cDNA library yielded 24 positive clones. The recombinant plasmids of each of these clones were extracted and sized by electrophoresis on 1 % agarose gel. Six of the plasmids were of similar size and contained a cDNA insert of 1000 bp corresponding to a full length TGH mRNA. Restriction analysis confirmed that all clones were identical, and one of these, designated ptiGH6, was chosen for further analysis. The entire cloned c-DNA fragment was transferred between the EcoRI-BamHI sites of the M13mp18 and M13mp19 vectors (Vieria and Messing, 1982), and its nucleotide sequence was determined using the dideoxynucleotide chain termination method of Sanger et al. (1977).

The nucleotide sequence of the TGH cDNA of ptiGH6 is presented in figure 7. The cDNA fragment has a length of 1000 nucleotides and contains an open reading frame of 621 nucleotides followed by a 3' untranslated region carrying a polyadenylation signal located 18 nucleotides upstream the poly (A) tail. The polyA tail is about 150bp long. The ATG initiator codon at nucleotide 36 represents the putative translational start site as a part of a consensus initiation sequence PCCATGP (Kozak, 1986). The open reading frame encodes a protein of 204 amino acid residues with a calculated molecular weight of 23,114 daltons (Figure 7). The hydropathy profile of the deduced amino acid sequence, determined using the algorithm described by Kyte and Doolittle (1982), shows that the NH2 terminus is highly hydrophobic and exhibits all characteristics of a signal peptide. This peptide signal is supposedly cleaved off during processing to give a mature protein of 187 AA residues with 4 cysteine residues.

We have compared the derived amino acid sequence of the TGH protein with the amino acid sequences of other known fish growth hormones such as tuna, yellow tail, trout and salmon. We observed a similarity of 89 % between the TGH and tuna GH and of 84,5 % between TGH and yellow tail GH, while only 66 % similarity was found with trout GH and salmon GH. These results are in agreement with the taxonomic classification of these fish. Tilapia, tuna and yellow tail belong to the order of perciform, while trout and salmon belong to the order of salmoniform. Furthermore, these sequence comparisons further support above conclusion that the first 17 amino acids of the TGH protein comprise the signal sequence and that the mature protein starts at glutamine residue 1.

Expression of the TGH gene

The same rationale as was used previously for the PRL genes, was used for expressing the TGH gene (figure 4). In a first step we constructed the modified 5' end of the TGH gene by combining a HindII-PvuI fragment of the gene starting at codon 15 with a synthetic oligonucleotide containing the initiator ATG followed by the first 14 codons of the mature TGH protein. In a second step we inserted the modified 5' end of the TGH gene on a NdeI-PvuI fragment together with the remaining PvuI-BamHI fragment of the TGH gene in the expression vector pARAE.

The construction of the plasmid pT7TGH diagrammed in figure 8 was performed as follows. In the first step we constructed the plasmid pTGHM containing the modified 5' end of the TGH gene by inserting the synthetic oligonucleotide described in figure 8 together with HincII-PvuI fragment into the plasmid pT7CTBR. pT7CTBR is a derivative of pARAE and like pARAE it possesses a unique NdeI site. In addition, it also contains a PvuII and PstI site between the promoter and the terminator. pT7CTBR was cleaved with NdeI and partially with PvuI and the large fragment was ligated together with the synthetic oligonucleotide and the HincII-PvuI fragment isolated from the plasmid pTGH. The ligation mixture was transformed into E. coli HB101 and transformants containing the desired recombinant plasmid pTGHM were identified using standard plasmid analysis techniques. In a second step we constructed the plasmid pT7TGH by inserting 2 fragments of the TGH gene, respectively the NdeI-SstI fragment from plasmid pTGHM (containing the modified 5' end of the tiGH gene) and the SstI-BamHI fragment from plasmid ptiGH (containing the rest of the TGH cDNA) into the expression vector pARAE. The two fragments were purified by agarose gel electrophoresis after digesting the two plasmids with the respective restriction enzymes and ligated together with the plasmid pARAE digested with the enzymes NdeI and BamHI. The ligation mixture was then used to transform E. coli HB101 and transformants containing the desired recombinant plasmid, designated pT7TGH, were identified using standard plasmid analysis techniques.

In order to express the TGH protein, the pT7TGH plasmid was introduced into the E. coli strain BL21 (DE3) described earlier. E. coli BL21 (DE3) containing the pT7TGH plasmid were grown overnight in 5ml Luria-broth in the presence of 100 μg/ml ampicillin. Of this culture, 0.4 ml were used to inoculate 10 ml of the same medium supplemented with ampicillin (100 μg/ml). and 0.4 mM IPTG was added when the culture reached $A_{600m}$ = 0.8. After three hours, the cells were harvested and lysed in SDS sample buffer (Laemmli, 1970) and analyzed by sodium dodecylsulfate polyacrylamide gel electrophoresis (SDS-PAGE) with Coomassie blue staining. Comparison of the total cell proteins in extracts of non-induced cultures and IPTG induced cultures shows that a new abundant 24 kd protein is produced.

The newly synthesized TGH protein is made in large amounts, reaching 25 % of the total cellular protein. Western blot analysis of the SDS PAGE using a salmon growth hormone antiserum shows that the newly synthesized proteins cross reacts with the antiserum, thus demonstrating that the TGH protein corresponds to the tilapia growth hormone.

The overproduced TGH protein is found to be contained within inclusion bodies, which are readily purified after lysing the cells by sonication and centrifugation. The growth hormone protein can be easily pruified by solubilizing the purified inclusion bodies in the presence of 8 M urea, whereafter the solution is dialyzed against buffers containing protease inhibitors. The protein is further purified by chromatography on Sephadex G100.


FIGURE LEGENDS


Figure 1 :


Restriction maps of the TPRL cDNA inserts :


(a) TPRLII cDNA and (b) TPRLI cDNA.

The boxes indicate the positions of the TPRL coding regions. The shaded boxes indicate the segment coding for the signal peptide while the open boxes indicate the segments coding for the mature prolactins. The vertical lines show the positions of the restriction enzyme clearage sites : Av = Aval; Ba = BamH1; Bc = Bcll; Bg = Bglll; Ec = EcoRl; Fo = Fokl; Hi = Hincll; Ps = Pstl; Pv = Pvull; Sc = Sacl; Sp = Sphl; Ta = Taql;


Figure 2 :

Nucleotide sequence of the TPRLI cDNA fragment of plasmid pTPRLI and amino acid sequence of the TPRLI protein derived from the nucleotide sequence. The amino acids of the mature TPRLI are numbered and the negatively numbered amino acids correspond to the signal peptide.

The total nucleotide sequence is numbered from (-50) to (x).

The coding sequence is numbered from (1) to (636).


Figure 2bis :

Nucleotide sequence of the modified PRLI cDNA fragment encoding the modified TPRLI protein containing the following amino acid substitutions : Leu at position 7 and Ser at position 100. The amino acids of the mature TPRLI are numbered and the negatively numbered amino acids correspond to the signal peptide.

The total nucleotide sequence is numbered from (-50) to (x).

The coding sequence is numbered from (1) to (636).


Figure 3 :

Nucleotide sequence to the PRLII cDNA fragment of plasmid pTPRLII and amino acid sequence of the TPRLII protein derived from the nucleotide sequence. The amino acids of the mature PRLII are numbered and the negatively numbered amino acids correspond to the signal peptide.

The total nucleotide sequence is numbered from (-38) to (y).

The coding sequence is numbered from (1) to (600).


Figure 4 :

General scheme for the expression of mature TPRL and TGH proteins in E. coli.

(a) Scheme for modifying the 5′ end of the gene so as to delete the signal peptide coding region (open box).

(b) Scheme for engineering modified genes into the bacteriophage T7 promoter (PT7) vectors.


Figure 5 :

Construction of the pT7TPRLI expression plasmid.

(a) Construction of the pARAE expression vector carrying the bacteriophage T7 promoter.

(b) Construction of the modified TPRLI gene inserted behind bacteriophage T7 promoter.


Figure 6 :

Construction of the pT7TPRLII expression vector.

(a) Construction of the modified 5′ end of the TPRLII gene.

(b) Reconstruction of the modified TPRLII gene behind the bacteriophage T7 promoter.


Figure 7 :

Nucleotide sequence of the TGH cDNA fragment of plasmid pTGH and amino acid sequence of the TGH protein derived from the nucleotide sequence. The amino acids of the mature TGH are numbered and the negatively numbered amino acids correspond to the signal peptide.

The total nucleotide sequence is numbered from (-26) to (z).

The coding sequence is numbered from (1) to (621).

Figure 8 :

Construction of the pT7TGH expression vector.
(a) Construction of the modified 5 end of the TGH gene.
(b) Construction of the modified TGH gene behind the bacteriophage T7 promoter.

## REFERENCES

- Agellon L.B. and Chen T.T., (1986). Rainbow trout growth hormone : molecular cloning of cDNA and expression in Escherichia coli. DNA 5, 463-471.
- Chirgwin J.M., Przybyla A.E., MacDonald R.J. and Rutter W.J., (1979) Isolation of biologically active ribonucleic acid from sources enriched in ribonuclease. Biochemistry 18, 5294-5299.
- Clarke W.C., (1973). Sodium-retaining bioassay of prolactin in the intact teleost Tilapia mossambica acclimated to sea water. Gen. Comp. Endocrinol. 21, 489-512.
- Clarke W.C. and Bern H.A., (1980). Comparative endocrinology of prolactin. In Li CH (ed) : "Hormonal Proteins and Peptides", New York : Academic Press 8, 105-197.
- Dharmamba M., (1970). Studies of the effects of hypophysectomy and prolactin on plasma osmolality and plasma sodium in Tilapia mossambica. Gen. Comp. Endocrinol. 14, 256-269.
- Dharmamba M. and Maetz J., (1972). Effects of hypophysectomy and prolactin on the sodium of Tilapia mossambica in fresh water. Gen. Comp. Endocrin. 19, 175-183.
- Dharmamba M. and Maetz J., (1976). Branchal sodium exchange in sea water - adapted Tilapia mossambica : effect of prolactin and hypophysectomy. J. Endocrinol. 70, 293-299. F
- Foskett J.K., Machen T.E. and Bern H.A., (1982). Chloride secretion and conductance of teleost opercular membrane : effects of prolactin. Am. J. Physiol. 242, R380-389.
- Gonzales-Villasenor L.I., Zhang P., Chen T.T. and Powers D.A., (1988). Molecular cloning and sequencing of coho salmon growth hormone cDNA. Gene 65, 239-246.
- Gubler U. and Hoffman B.J., (1983). A simple and very efficient method for generating cDNA libraries. Gene 25, 263-269.
- Hirano T., (1986). The spectrum of prolactin action in teleosts. In : "Comparative Endocrinology Developments and Directions". C.L. Ralph, ed. (Alan R. Liss, Inc. : N.Y.), pp. 53-74.
- Kozak M., (1986). Point mutations define a sequence flanking the AUG initiator codon that modulates translating by eukaryotic ribosomes. Cell 44, 283-292.
- Kyte J. and Doolittle R.F., (1982). A simple method for displaying the hydrophatic character of a protein. J. Mol. Biol. 157, 105-132.
- Laemmli U.K., (1970). Cleavage of structural proteins during the assembly of the head of bacteriophage T4. Nature (London), 227, 680-685.
- Loretz C.A. and Bern H.A., (1982). Prolactin and osmoregulation in vertebrates. Neuroendocrinology 35, 292 304.
- Mandel M. and Higa A., (1970). Calcium dependent bacteriophage DNA infection. J. Mol. Biol. 53, 159-162.
- Maniatis T., Fritsch E.F. and Sambrook J.. J. "Molecular Cloning", Cold Spring Harbor Laboratory, 1982.
- Mercier L., Rentier-Delrue F., Swennen D., Lion R., Le Goff P., Prunet P. and Martial J.A. Rainbow trout prolactin cDNA cloning in E. coli. (1989). DNA 8, in press.
- Nicoll C.S., Steiny S.S., Kind D., Nishioka R.S., Mayer G.L., Eberhardt N.L., Baxter J.D., Yamanaka M.K., Miller J.A., Seilhamer J.J., Schilling J.W. and Johnson L.K. (1987). The primary structure of coho salmon growth hormone and its cDNA. Gen. Comp. Endocrinol. 68, 387-399.
- Rosenberg A.H., Lade B.N., Chin D., Lin S., Dunn J.J. and Studier F.W., (1987). Vectors for selective expression of cloned cDNAs by T7 RNA polymerase. Gene 56, 125-135.
- Sanger F., Nickler S. and Coulson A.R., (1977). DNA sequencing with chain-terminating inhibitors. Proc. Natl. Acad. Sci. USA 74, 5463-5467.
- Sato N., Watanabe K, Murata K., Sabaguchi M., Kariya Y., Kimura S., Nonaka M. and Kimura A., (1988). Molecular cloning and nucleotide sequence of tuna growth hormone cDNA. Biochem. Biophys. Acta 949, 35-42.
-Sekine S., Mizukami T., Nishi T., Kuwana Y., Saito A., Sato M., Itoh S. and Kawauchi H., (1985). Cloning and expression of cDNA for salmon growth hormone in Escherichia coli. Proc. Natl. Acad. Sci. USA 82, 4306-4310.
- Song S., Trinh K.Y., Hew C.L., Hwang S.J., Belkhode S. and Idler D.R., (1988). Molecular cloning and expression of salmon prolactin cDNA. Eur. J. Biochem. 172, 279-285.
- Specker J.L., King D.S., Nishioka R.S., Shirahata K., Yamaguchi K. and Bern H.A., (1985). Isolation and partial characterization of a pair of prolactins released in vitro by the pituitary of a cichled fish, oreochromis mossambicus. Proc. Natl. Acad. Sci. USA 82, 7490-7494.

- Studier F.W. and Moffat B.A., (1986). Use of bacteriophage T7 RNA polymerase to direct selective high-level expression of cloned genes. J. Mol. Biol. 186,

- Prunet and Bornancin, (1988) 113-130.

- Ullrich A., Shine J., Chirgwin J., Pictet R., Tischer E., Rutter W.J. and Goodman H.M., (1977). Rat insulin genes : construction of plasmids containing the coding sequences. Science 196 : 1313-1319.

- Viera J. and Messing J., (1982). The pUC plasmids, an M13mp7 derived system for insertion mutagenesis and sequencing with synthetic universal primers. Gene 19, 259-268.

- Yamagushi K., Specker J.F., King D.S., Yokoo Y., Nishioka R.S., Hirano T. and Bern H.A., (1988). Complete amino acid sequences of a pair of fish (Tilapia) prolactins, tPRL$_{177}$ and tPRL$_{188}$. The J. Biol. Chem. 263, 9113-9121.

- Yanish-Perron C., Vieira J. and Messing J., (1985). Improved M13 phage cloning vectors and host strains : nucleotide sequences of the M13mp18 and pUC19 vectors. Gene 33, 103-119.

- Yasuda A., Miyazima K.I., Kawauchi H., Peter R.E., Lin H.R., Yamaguchi K. and Sano H. (1987). Primary structure of common carp prolactins. Gen. Comp. Endocrinol. 66, 280-290.

- Young P.S., McCormick S.D., Demarest J.R., Lin R.J., Nishioka R.S. and Bern H.A., (1988). Effect of salinity, hypophysectomy and prolactin on whole animal trasepithelial potentials in the Tilapia Oreochromis Mossambicus. Gen. Comp. Endocrinol. 71, 389-397.

## Claims

1. Process for preparing a stable recombinant polypeptide presenting growth and/or osmoregulatory properties which comprises the following steps :

- a cellular host, which is previously transformed by an appropriate vector containing a nucleic acid coding for polypeptides having

. the polypeptide sequence of the natural growth promoting and/or osmoregulatory hormones of tilapia or catfish or

. a polypeptide sequence containing part or the totality of the sequence of the natural growth promoting and/or osmoregulatory hormones of tilapia or brill, in so far as the polypeptide sequence produced has growth promoting and/or osmoregulatory properties,

is cultivated in an appropriate medium at a temperature from about 28°C to about 42°C depending on the selected microorganism and

- the polypeptide produced by said transformed cellular host is recovered from the above said medium culture and, if need be, purified.

2. Process according to claim 1 wherein the nucleic acids coding for the natural polypeptides presenting growth promoting and/or osmoregulatory properties are under the control of regulatory elements, such as a promoter recognized by the RNA polymerase of the host organism, a translation initiation and terminatory signal.

3. Process according to anyone of claims 1 or 2, wherein the cellular host is E. coli, the growth of which is stopped at the exponential phase and the polypeptide produced by said transformed cellular host is recovered from the medium culture free from other proteins and exocellular enzymes liable to be secreted by E. coli.

4. Process according to anyone of claims 1 or 2, wherein the cellular host is yeast.

5. Process according to anyone of claims 1 to 4, wherein the recombinant polypeptide is preceded by a signal peptide, particularly by a signal peptide having the same amino acid sequence as the natural signal peptide in tilapia or catfish.

6. Process according to anyone of claims 1 to 4, wherein the recombinant polypeptide is preceded by a methionine.

7. Process according to anyone fo claims 1 to 6 wherein the recombinant polypeptide comprises in its polypeptide chain an aminoacid sequence containing in one of the following amino sequences :

* from amino acid (-24) to amino acid (188) represented in Fig. 2,
* from amino acid (-24) to amino acid (188) represented in fig. 2bis,
* from amino acid (-23) to amino acid (177) represented in Fig. 3,
* from amino acid (-20) to amino acid (187) represented in Fig. 7,
* from amino acid (1) to amino acid (188) represented in Fig. 2,
* from amino acid (1) to amino acid (188) represented in Fig. 2, preceded by Met,
* from amino acid (1) to amino acid (188) represented in fig. 2bis,
* from amino acid (1) to amino acid (188) represented in fig. 2bis, preceded by Met,
* from amino acid (1) to amino acid (177) represented in Fig. 3,
* from amino acid (1) to amino acid (177) represented in Fig. 3, preceded by Met,
* from amino acid (1) to amino acid (187) represented in Fig. 7,
* from amino acid (1) to amino acid (187) represented in Fig. 7, preceded by Met,

or comprises one of the above amino acid sequences,

or is constituted by one of the above mentioned amino acid sequences.

8. Process according to claim 7 wherein the recombinant polypeptide comprises in its polypeptide chain an amino acid sequence
- contained in one of the following sequences :
* from amino acid (-20) to amino acid (187) represented in Fig. 7,
* from amino acid (1) to amino acid (187) represented in Fig. 7,
* from amino acid (1) to amino acid (187) represented in Fig. 7, preceded by Met,
- or comprises one of the abovesaid amino acid sequence or constituted by one of the abovesaid amino acid sequences,
- or comprises or is constituted by one of the following amino acid sequences :
* from amino acid (-24) to amino acid (188) represented in Fig. 2,
* from amino acid (-24) to amino acid (188) represented in Fig. 2 bis,
* from amino acid (-23) to amino acid (177) represented in fig. 3,
* from amino acid (1) to amino acid (188) represented in fig. 2,
* from amino acid (1) to amino acid (188) represented in Fig. 2, preceded by Met,
* from amino acid (1) to amino acid (188) represented in Fig. 2 bis, preceded by Met, * from amino acid (1) to amino acid (177) represented in Fig. 3, preceded by Met.

9. Process according to claim 8, wherein the recombinant polypeptide comprises in its polypeptide chain an amino acid sequence contained in one of the following sequences :
* from amino acid (1) to amino acid (188) represented in fig. 2bis,
* from amino acid (1) to amino acid (177) represented in Fig. 3,
or comprises one of the above amino acid sequences or is constituted by one of the above mentioned amino acid sequences.

10. Recombinant polypeptide comprising in its polypeptidic chain, an amino acid sequence
- contained in one of the following sequences :
* from amino acid (-20) to amino acid (187) represented in Fig. 7,
* from amino acid (1) to amino acid (187) represented in Fig. 7,
* from amino acid (1) to amino acid (187) represented in Fig. 7, preceded by Met,
- or comprises one of the abovesaid amino acid sequence or constituted by one of the abovesaid amino acid sequences,
- or comprises or is constituted by one of the following amino acid sequences :
* from amino acid (-24) to amino acid (188) represented in Fig. 2,
* from amino acid (-24) to amino acid (188) represented in Fig. 2 bis,

* from amino acid (-23) to amino acid (177) represented in fig. 3,
* from amino acid (1) to amino acid (188) represented in fig. 2,
* from amino acid (1) to amino acid (188) represented in Fig. 2, preceded by Met,
* from amino acid (1) to amino acid (188) represented in Fig. 2 bis, preceded by Met,
* from amino acid (1) to amino acid (177) represented in Fig. 3, preceded by Met.

11. Nucleic acid comprising a nucleotidic chain coding for the polypeptides according to claims 10.

12. Nucleic acid comprising a part or the totality of one of the following nucleotidic sequences :
* from nucleotide (-26) to nucleotide (z) of Fig. 7,
* from nucleotide (1) to nucleotide (621) of figure 7,
* from nucleotide (61) to nucleotide (621) of Fig. 7,
* from nucleotide (61) to nucleotide (621) of Fig. 7, preceded by the AUG codon,
or is constituted by one of the above mentioned nucleotidic sequences.

13. Nucleic acid comprising one of the following nucleotidic sequences :
* from nucleotide (-50) to nucleotide (x) of fig.2,
* from nucleotide (1) to nucleotide (636) of fig.2,
* from nucleotide (73) to nucleotide (636) of fig. 2,
* from nucleotide (73) to nucleotide (636) of fig. 2, preceded by the AUG codon,
* from nucleotide (-38) to nucleotide (y) of fig. 3,
* from nucleotide (1) to nucleotide (600) of fig. 3,
* from nucleotide (70) to nucleotide (600) of fig.3,
* from nucleotide (70) to nucleotide (600) of fig.3, preceded by the AUG codon,
* from nucleotide (-50) to nucleotide (x) of Fig. 2bis,
* from nucleotide (1) to nucleotide (636) of Fig. 2 bis,
* from nucleotide (73) to nucleotide (636) of Fig. 2 bis,
* from nucleotide (73) to nucleotide (636) of Fig. 2 bis, preceded by the AUG codon,
or constituted by one of the above mentioned nucleotidic sequences.

14. Recombinant vector, particularly for the cloning and/or expression, particularly of the plasmid, cosmid or phage type, containing a nucleic acid coding for a natural polypeptide - presenting growth promoting and/or osmoregulatory properties -of tilapia or catfish in particular in one of its site which is not essential for its replication, and in particular containing the nucleic acids coding for one of the following polypeptides :
* from amino acid (-24) to amino acid (188) represented in Fig. 2,
* from amino acid (-24) to amino acid (188) represented in fig. 2bis,
* from amino acid (-23) to amino acid (177) represented in Fig. 3,
* from amino acid (-20) to amino acid (187) represented in Fig. 7,

* from amino acid (1) to amino acid (188) represented in Fig. 2,

* from amino acid (1) to amino acid (188) represented in Fig. 2, preceded by Met,

* from amino acid (1) to amino acid (188) represented in fig. 2bis,

* from amino acid (1) to amino acid (188) represented in fig. 2bis, preceded by Met,

* from amino acid (1) to amino acid (177) represented in Fig. 3,

* from amino acid (1) to amino acid (177) represented in Fig. 3, preceded by Met,

* from amino acid (1) to amino acid (187) represented in Fig. 7,

* from amino acid (1) to amino acid (187) represented in Fig. 7, preceded by Met,

15. Recombinant vector according to claim 14 containing

- regulatory elements necessary to allow the expression of a sequence of amino acids of a polypeptide presenting growth promoting and/or osmoregulatory properties of tilapia or catfish in a cellular host and

- possibly a promoter, particularly an inducible promoter.

16. Cellular host which is transformed by a recombinant vector according to any one of claim 14 and 15 and comprising the regulation elements enabling the expression of the nucleic sequence coding for the polypeptide presenting growth promoting and/or osmoregulatory properties - of tilapia or catfish in this host.

17. Cellular host according to claim 16 which is E. Coli transformed by a the vector according to claim 14 or 15.

18. Expression product of a nucleic acid expressed by a transformed cellular host according to claim 16 or 17.

19. Composition for fish containing at least one of the polypeptides such as the ones obtained according to the process of anyone of claims 1 to 9, or mixtures thereof in an efficient amount such as 5 ng to 1 μg of polypeptide / g of fish per day, when administered by injection.

20. Food for fish, containing besides the classical nutrient ingredients, at least anyone of the polypeptides such as the ones obtained according to anyone of claims 1 to 9, in an efficient amount such as 1 μg to 1 mg of polypeptide / g of fish food per day.

FIGURE I

FIGURE II

```
        GA TCC CCC GAG GAG AAG AAG CAA CAG CTA ACG GAC AGA AAT AGA AGA
-50

       -24                    -20                           -10
       Met Ala Gln Arg Arg Thr Ser Gly Thr Asn Leu Phe Met Thr Val
       GAG ATG GCT CAG AGA AGA ACC AGT GGA ACC AAC CTC TTC ATG ACA GTG
       1
                                               -1   1
       Leu Cys Val Val Ala Met Cys Arg Ala Val Pro Ile Asn Glu Leu Phe
       TTG TGT GTG GTG GCA ATG TGC AGA GCC GTC CCC ATC AAC GAA CTG TTC
                                           73
                 10                                     20
       Glu Arg Ala Ser Gln His Ser Asp Lys Leu His Ser Leu Ser Thr Thr
       GAG CGA GCC TCT CAG CAC TCT GAC AAA CTG CAC TCT CTC AGC ACG ACG

                           30
       Leu Thr Gln Glu Leu Asp Ser His Phe Pro Pro Ile Gly Arg Val Ile
       CTC ACC CAG GAG CTG GAC TCT CAC TTC CCT CCT ATA GGC AGG GTG ATC

        40                                    50
       Met Pro Arg Pro Ala Met Cys His Thr Ser Ser Leu Gln Thr Pro Ile
       ATG CCG CGG CCT GCA ATG TGC CAC ACC TCC TCT CTA CAG ACG CCC ATT

                           60                                    70
       Asp Lys Asp Gln Ala Leu Gln Val Ser Glu Ser Asp Leu Met Ser Leu
       GAC AAG GAC CAA GCA CTT CAA GTG TCA GAG TCA GAT TTG ATG TCC CTG

                                       80
       Ala Arg Ser Leu Leu Gln Ala Trp Ser Asp Pro Leu Val Val Leu Ser
       GCT CGC TCC CTC CTC CAA GCC TGG TCA GAC CCT CTC GTA GTC CTG TCC

                 90                                    100
       Ser Ser Ala Ser Thr Leu Pro His Pro Ala Gln Ser Thr Ile Phe Asn
       TCC TCC GCT AGT ACC CTG CCT CAC CCA GCA CAA AGC ACC ATA TTC AAC

                           110
       Lys Ile Gln Glu Met Gln Gln Tyr Ser Lys Ser Leu Lys Asp Gly Leu
       AAG ATC CAG GAG ATG CAG CAG TAC TCC AAA AGT CTC AAG GAT GGC CTG

       120                                   130
       Asp Val Leu Ser Ser Lys Met Gly Ser Pro Ala Gln Ala Ile Thr Ser
       GAT GTA CTA TCT AGT AAG ATG GGT TCA CCT GCT CAG GCC ATC ACT TCA

                           140                                   150
       Leu Pro Tyr Arg Gly Gly Thr Asn Leu Gly His Asp Lys Ile Thr Lys
       CTG CCC TAC CGA GGA GGC ACC AAC CTC GGC CAT GAC AAG ATC ACC AAG

                                       160
       Leu Ile Asn Phe Asn Phe Leu Leu Ser Cys Leu Arg Arg Asp Ser His
       TTG ATC AAC TTC AAC TTT CTG CTG TCC TGC CTC CGT CGG GAC TCT CAC

                 170                                   180
       Lys Ile Asp Ser Phe Leu Lys Val Leu Arg Cys Arg Ala Ala Lys Met
       AAG ATT GAC AGC TTC CTG AAA GTC CTG CGC TGC CGG GCA GCA AAG ATG

                 188
       Gln Pro Glu Met Cys ***
       CAA CCA GAG ATG TGC TGA AGA GTG GGT GTC CAG CCT GAC TCT GTG GGG
                 636
```

FIGURE II (suite)

AGA TTG TTC TGA AAG CTT GTA TTG CCT ATA GAA TTA TAC ATG GTA CTA

TAT CCC ACC TGC TGA TTA GTT TTG GAA CAC GAG GAT AAG GAG GAG TTA

TAA GGT ACA AAA ACA CAT AAA AAT TGA CCT TCT TGC TCC GTT TCT CAG

TGT GTA TGT ATA AAT TAA AGC TGC TTT CAG CTG TTG CCT TAT CTC AAG

GGG TTA CCA CAG TGG GCC ACT CCC CAT GTT TAA ATG TGT TTT ATG CTG

GAT GTC CTT CCT GAT GCA ACC CCA AAG AAG TTT GTG TCT CCT CCT GGG

ATT ATG CCA GGA GAT CTT TTG CTT GTT TAT GCA CAT GTG CAA ACC TCT

ACA CTA TGG AGG ATT GTT AAA TAG TAG CCT TAA AGG TTA ACA GAT TTA

AAG TAT AGG AAG GGG AGC TGC AGG CTG TTG TCC GAC CTT ATG ATG GAG

ATG GAG CAC CTA CCA ATT TTT ATT TCC AGC TGG CCT GGC ATT ATG AAG

TGA CCA CAC GGT GGC CAC GAA TGG AAG CAA ATC AGT GTG TCA GCC TGT

GAC TCC TCC TCA TCC TTT TGT TCT GTT GAA GCA GTA TCC CCA CAA AGT

CAT ACT ACT AAT GGA GAA AAT GAG TGG AGA ATG TTT TCT TCC TAA CTA

ATG CTT AAT TAA CTG CAG CAT TTG CAC TTT AGA TTA AAT GAC ATC TTT

TAA AAA GCA TTG TTA CCT CAG TGC CTT TTA ACT GAA CTG TTT CTT TTA

AAT CTG TAT TTC AAT AAA GTT TTT CTT GCA CAG TAA AAA AAA AAA AAA

AAA AAA AAA AAA AAA AAA AAA AAA AAA

x

FIGURE II BIS

```
    GA TCC CCC GAG GAG AAG AAG CAA CAG CTA ACG GAC AGA AAT AGA AGA
- 50
     -24                    -20                                       -10
    Met Ala Gln Arg Arg Thr Ser Gly Thr Asn Leu Phe Met Thr Val
GAG ATG GCT CAG AGA AGA ACC AGT GGA ACC AAC CTC TTC ATG ACA GTG
    1
                                              -1   1
Leu Cys Val Val Ala Met Cys Arg Ala Val Pro Ile Asn Glu Leu Leu
TTG TGT GTG GTG GCA ATG TGC AGA GCC GTC CCC ATC AAC GAA CTG TTC
                                              73
        10                                            20
Glu Arg Ala Ser Gln His Ser Asp Lys Leu His Ser Leu Ser Thr Thr
GAG CGA GCC TCT CAG CAC TCT GAC AAA CTG CAC TCT CTC AGC ACG ACG

                        30
Leu Thr Gln Glu Leu Asp Ser His Phe Pro Pro Ile Gly Arg Val Ile
CTC ACC CAG GAG CTG GAC TCT CAC TTC CCT CCT ATA GGC AGG GTG ATC

    40                                            50
Met Pro Arg Pro Ala Met Cys His Thr Ser Ser Leu Gln Thr Pro Ile
ATG CCG CGG CCT GCA ATG TGC CAC ACC TCC TCT CTA CAG ACG CCC ATT

                        60                                    70
Asp Lys Asp Gln Ala Leu Gln Val Ser Glu Ser Asp Leu Met Ser Leu
GAC AAG GAC CAA GCA CTT CAA GTG TCA GAG TCA GAT TTG ATG TCC CTG

                                80
Ala Arg Ser Leu Leu Gln Ala Trp Ser Asp Pro Leu Val Val Leu Ser
GCT CGC TCC CTC CTC CAA GCC TGG TCA GAC CCT CTC GTA GTC CTG TCC

        90                                            100
Ser Ser Ala Ser Thr Leu Pro His Pro Ala Gln Ser Ser Ile Phe Asn
TCC TCC GCT AGT ACC CTG CCT CAC CCA GCA CAA AGC ACC ATA TTC AAC

                        110
Lys Ile Gln Glu Met Gln Gln Tyr Ser Lys Ser Leu Lys Asp Gly Leu
AAG ATC CAG GAG ATG CAG CAG TAC TCC AAA AGT CTC AAG GAT GGC CTG

120                                            130
Asp Val Leu Ser Ser Lys Met Gly Ser Pro Ala Gln Ala Ile Thr Ser
GAT GTA CTA TCT AGT AAG ATG GGT TCA CCT GCT CAG GCC ATC ACT TCA

                        140                                    150
Leu Pro Tyr Arg Gly Gly Thr Asn Leu Gly His Asp Lys Ile Thr Lys
CTG CCC TAC CGA GGA GGC ACC AAC CTC GGC CAT GAC AAG ATC ACC AAG

                                160
Leu Ile Asn Phe Asn Phe Leu Leu Ser Cys Leu Arg Arg Asp Ser His
TTG ATC AAC TTC AAC TTT CTG CTG TCC TGC CTC CGT CGG GAC TCT CAC

        170                                            180
Lys Ile Asp Ser Phe Leu Lys Val Leu Arg Cys Arg Ala Ala Lys Met
AAG ATT GAC AGC TTC CTG AAA GTC CTG CGC TGC CGG GCA GCA AAG ATG

        188
Gln Pro Glu Met Cys ***
CAA CCA GAG ATG TGC TGA AGA GTG GGT GTC CAG CCT GAC TCT GTG GGG
        636
```

EP 0 387 457 A1

FIGURE II BIS (suite)

AGA TTG TTC TGA AAG CTT GTA TTG CCT ATA GAA TTA TAC ATG GTA CTA

TAT CCC ACC TGC TGA TTA GTT TTG GAA CAC GAG GAT AAG GAG GAG TTA

TAA GGT ACA AAA ACA CAT AAA AAT TGA CCT TCT TGC TCC GTT TCT CAG

TGT GTA TGT ATA AAT TAA AGC TGC TTT CAG CTG TTG CCT TAT CTC AAG

GGG TTA CCA CAG TGG GCC ACT CCC CAT GTT TAA ATG TGT TTT ATG CTG

GAT GTC CTT CCT GAT GCA ACC CCA AAG AAG TTT GTG TCT CCT CCT GGG

ATT ATG CCA GGA GAT CTT TTG CTT GTT TAT GCA CAT GTG CAA ACC TCT

ACA CTA TGG AGG ATT GTT AAA TAG TAG CCT TAA AGG TTA ACA GAT TTA

AAG TAT AGG AAG GGG AGC TGC AGG CTG TTG TCC GAC CTT ATG ATG GAG

ATG GAG CAC CTA CCA ATT TTT ATT TCC AGC TGG CCT GGC ATT ATG AAG

TGA CCA CAC GGT GGC CAC GAA TGG AAG CAA ATC AGT GTG TCA GCC TGT

GAC TCC TCC TCA TCC TTT TGT TCT GTT GAA GCA GTA TCC CCA CAA AGT

CAT ACT ACT AAT GGA GAA AAT GAG TGG AGA ATG TTT TCT TCC TAA CTA

ATG CTT AAT TAA CTG CAG CAT TTG CAC TTT AGA TTA AAT GAC ATC TTT

TAA AAA GCA TTG TTA CCT CAG TGC CTT TTA ACT GAA CTG TTT CTT TTA

AAT CTG TAT TTC AAT AAA GTT TTT CTT GCA CAG TAA AAA AAA AAA AAA

AAA AAA AAA AAA AAA AAA AAA AAA AAA

x

FIGURE III

```
                                                                    -23
                                                            Met Ala Gln
       CC CCG GGT ACC GAG CTC GAA TTC GAG CTC GCC CGA GAG ATG GCT CAG
- 38
-20
Arg Arg Ile Ser Gly Ser Lys Leu Met Met Val Leu Cys Val Val Ala
AGA AGA ATC AGT GGA AGC AAA CTC ATG ATG GTG TTG TGT GTG GTG GCA


                 -1   1                                      10
Met Cys Arg Ala Val Pro Ile Asn Asp Leu Ile Tyr Arg Ala Ser Gln
ATG TGC AGA GCC GTC CCC ATC AAC GAC CTC ATC TAT CGA GCC TCT CAA
                 70
                                        20
Gln Ser Asp Lys Leu His Ala Leu Ser Thr Met Leu Thr Gln Glu Leu
CAG TCT GAC AAA CTG CAC GCG CTC AGC ACG ATG CTC ACC CAG GAG CTG

       30                                               40
Gly Ser Glu Ala Phe Pro Ile Asp Arg Val Leu Ala Cys His Thr Ser
GGC TCT GAA GCA TTC CCT ATA GAC AGG GTT CTC GCG TGC CAC ACC TCC

                                50                              60
Ser Leu Gln Thr Pro Thr Asp Lys Glu Gln Ala Leu Gln Val Ser Glu
TCT CTA CAG ACG CCC ACT GAC AAG GAG CAA GCG CTT CAA GTA TCA GAG

                                        70
Ser Asp Leu Leu Ser Leu Ala Arg Ser Leu Leu Gln Ala Trp Ser Asp
TCG GAT CTG TTG TCC CTG GCT CGC TCC CTC CTG CAG GCT TGG TCG GAC

              80                                        90
Pro Leu Glu Val Leu Ser Ser Ser Thr Asn Val Leu Pro Tyr Ser Ala
CCT CTT GAA GTC CTG TCC TCC TCC ACT AAC GTC CTC CCT TAC TCA GCC

                                100
Gln Ser Thr Leu Ser Lys Thr Ile Gln Lys Met Gln Glu His Ser Lys
CAA AGC ACC TTA TCC AAA ACG ATC CAG AAG ATG CAG GAG CAC TCC AAA

       110                                       120
Asp Leu Lys Asp Gly Leu Asp Ile Leu Ser Ser Lys Met Gly Pro Ala
GAC CTC AAG GAT GGC CTG GAT ATA CTA TCT AGC AAG ATG GGT CCA GCT

                         130                                    140
Ala Gln Thr Ile Thr Ser Leu Pro Phe Ile Glu Thr Asn Glu Ile Gly
GCT CAG ACC ATC ACT TCA CTT CCA TTC ATA GAA ACC AAT GAG ATC GGC

                                        150
Gln Asp Lys Ile Thr Lys Leu Leu Ser Cys Phe Arg Arg Asp Ser His
CAG GAC AAG ATT ACC AAA CTG CTG TCC TGC TTT CGC CGG GAC TCT CAC

       160                                       170
Lys Ile Asp Ser Phe Leu Lys Val Leu Arg Cys Arg Ala Ala Asn Met
AAG ATC GAC AGC TTC TTG AAA GTC CTG CGC TGC CGG GCA GCA AAC ATG

              177
Gln Pro Gln Val Cys ***
CAA CCC CAG GTG TGC TGA AGA GTG GGT GTC CAG CCT CAC TCT GTG AAA
              600

AGA TCG TTC TTA AAG CTT CTG TTG CTT ACC AAA TAG AGA ATT GCT CAT
```

EP 0 387 457 A1

FIGURE III (suite)

GCT AGA AAT TTG CTG TCT TAG ATA TTA TCT GGT CTG CTA ATT AGT AAG

CTT TTT AGG CTT CAG GGA TTA AGG ATA AGG AGG AAT TAT AAA GCA TGC

ACA CAG GAA ACT ATC CAT GGT TTG GCT CTT TTA ACA CAG TGA GTA AAA

CAG TGA TCT TAC TTT CCA CTG AGC TAA ATC TGC GTG TAA AAT CTC TGT

GAC TCC TCA TTG TCT TTT CCT TCT GTT GCA GCA GAA ACC CCT GAA AGC

TAA TGT ACT AAA GCT GAA ATC TAG GGC TTC GAT GAA GAG TCA TGT TTC

TTC CTA ACT AAT GCT TAG TTG ATG AGA AAC TGC AGC ATT TGC ACT TGA

GTC GTC TCT TTT AAA AAG CGT TGT GAA TTC AGT GCC TTA TAA GTA AAC

TTT TTC TTT AAA ATC TGT ATT TCA ATA AAA AAA AAT CTT GCA CGG TGA

AAA AAA AAA AAA AAA A
                   y

Figure IV

EP 0 387 457 A1

FIGURE V

Figure VI

EP 0 387 457 A1

## FIGURE VII

```
                                          -20
                                          Met Pro Ala Met Asn Ser Val
  CT CGC CCG CAA ACA GAG CCT GAA CTG ATG CCA GCC ATG AAC TCA GTC
-26                                       1

              -10                                        -1   1
Val Leu Leu Leu Ser Val Val Cys Leu Gly Val Ser Ser Gln Gln Ile
GTC CTC CTG CTG TCG GTT GTG TGT TTG GGC GTC TCC TCT CAG CAG ATC
                                                         61

                      10
Thr Asp Ser Gln Arg Leu Phe Ser Ile Ala Val Asn Arg Val Thr His
ACA GAC AGC CAG CGT TTG TTC TCC ATT GCA GTC AAC AGA GTC ACG CAC

  20                                           30
Leu His Leu Leu Ala Gln Arg Leu Phe Ser Asp Phe Glu Ser Ser Leu
CTG CAC CTG CTC GCC CAG AGA CTC TTC TCG GAC TTT GAG AGC TCT CTG

                      40                                        50
Gln Thr Glu Glu Gln Arg Gln Leu Asn Lys Ile Phe Leu Gln Asp Phe
CAG ACG GAG GAG CAA CGT CAG CTC AAC AAA ATC TTC CTG CAG GAC TTC

                                              60
Cys Asn Ser Asp Tyr Ile Ile Ser Pro Ile Asp Lys His Glu Thr Gln
TGC AAC TCT GAT TAC ATC ATC AGC CCG ATC GAC AAA CAC GAG ACG CAG

           70                                            80
Arg Ser Ser Val Leu Lys Leu Leu Ser Ile Ser Tyr Gly Leu Val Glu
CGC AGC TCG GTC CTG AAG CTG CTG TCG ATC TCC TAT GGA CTG GTT GAG

                              90
Ser Trp Glu Phe Pro Ser Arg Ser Leu Ser Gly Gly Ser Ser Leu Arg
TCC TGG GAG TTT CCC AGT CGC TCT CTG TCT GGA GGT TCC TCT CTG AGG

100                                           110
Asn Gln Ile Ser Pro Arg Leu Ser Glu Leu Lys Thr Gly Ile Leu Leu
AAC CAG ATT TCA CCA AGG CTG TCT GAG CTT AAA ACG GGA ATC TTG CTG

                      120                                       130
Leu Ile Arg Ala Asn Gln Asp Glu Ala Glu Asn Tyr Pro Asp Thr Asp
CTG ATC AGG GCC AAT CAG GAT GAA GCA GAG AAT TAT CCT GAC ACC GAC

                                      140
Thr Leu Gln His Ala Pro Tyr Gly Asn Tyr Tyr Gln Ser Leu Gly Gly
ACC CTC CAG CAC GCT CCT TAC GGA AAC TAT TAT CAA AGT CTG GGA GGC

              150                                        160
Asn Glu Ser Leu Arg Gln Thr Tyr Glu Leu Leu Ala Cys Phe Lys Lys
AAC GAA TCG CTG AGA CAA ACT TAT GAA TTG CTG GCT TGC TTC AAG AAG

                          170
Asp Met His Lys Val Glu Thr Tyr Leu Thr Val Ala Lys Cys Arg Leu
GAC ATG CAC AAG GTG GAG ACC TAC CTG ACG GTA GCT AAA TGT CGA CTC

180                           187
Ser Pro Glu Ala Asn Cys Thr Leu
TCT CCA GAA GCA AAC TGC ACT CTG TAG CTC CAC CTA ATA TTG ATA CTG
                              621


ATA CGT GCT CTG TAG CCC CAC CCT CAT GTT GGC AAA CTC TGC TTA CAT
```

## FIGURE VII (suite)

GTG TTA GCA TTA GCA ATA GGA TAA TAA TAG CAG TGG TAA TCG TGA CAT

CAG AAC GTT TTT TCT GAC ATA ACT GTG ATG CAA GGT GTG AAC GGG AAT

AAT GTT ATC TGT GAA ATA AAT GTG TTG CAT TGA AAA AAA AAA AAA AAA

AAA AAA AAA AAA AAA AAA AAA AAA

z

FIGURE VIII

NdeI
TATGCAGCAGATCACAGACAGCCAGCGTTT
ACGTCGTCTAGTGTCTGTCGGTCGCAAA

GTTCTCCATTGCAGTC  HincII
CAAGAGGTAACGTCAG

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | CHEMICAL ABSTRACTS, vol. 87, no. 19, November 7, 1977, page 334, ref.no. 149071c; Columbus, Ohio, US W.C. CLARKE et al.: "Effect of teleost pituitary growth hormone on growth of Tilapia mossambica and on growth and seawater adaptation of sockey salmon (Oncorhynchus nerka)" &. GEN. COMP. ENDOCRINOL., 1977, 33(2), 174-8 | | C 12 N 15/16 C 12 N 15/18 C 07 K 13/00 C 12 P 21/02 C 12 N  1/21 A 23 K  1/165// |
| | * Abstract * | 19,20 | (C 12 N  1/21 C 12 R  1:19) |
| Y | | 1-8,10-18 | |
| Y | EP-A-0 239 075 (KYOWA HAKKO KOGYO CO., LTD) | | |
| | * Whole document * | 1-8,10-20 | |
| | | | **TECHNICAL FIELDS SEARCHED (Int. Cl.4)** |
| Y,D | BIOCHEM. BIOPHYS. ACTA, vol. 949, 1988, pages 35-42; Elsevier Sc. Publ. BV N. SATO et al.: "Molecular cloning and nucleotide sequence of tuna growth hormone cDNA" | | C 12 N C 07 K C 12 P |
| | * Whole article * | 1-8,10-20 | |
| Y | PROC. NATL. ACAD. SCI. USA, vol. 82, November 1985, pages 7490-7494; US J.L. SPECKER et al.: "Isolation and partial characterization of a pair of prolactins released in vitro by the pituitary of a cichlid fish, Oreochromis mossambicus" ./. | | |

The present search report has been drawn up for all claims

| Place of search | Date of completion of the search | Examiner |
|---|---|---|
| THE HAGUE | 06-12-1989 | DECAMPS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82

## DOCUMENTS CONSIDERED TO BE RELEVANT

| Category | Citation of document with indication. where appropriate. of relevant passages | Relevant to claim | CLASSIFICATION OF THE APPLICATION (Int. Cl 4) |
|---|---|---|---|
| | * Page 7490, column 1, last paragraph; page 7492, figure 2a * | 1-8,10-20 | |
| X,D | JOURNAL OF BIOLOGICAL CHEMISTRY, vol. 263, no. 19, July 5, 1988, pages 9113-9121; Am. Soc. for Biochemistry and Molecular Biology, US K. YAMAGUCHI et al.: "Complete amino acid sequences of a pair of fish (tilapia) prolactins, tPRL177 and tPRL188" | | |
| | * Whole article * | 19,20 | |
| Y | | 1-11, 13-18 | |
| Y | EP-A-0 201 882 (KYOWA HAKKO KOGYO CO., LTD) | | |
| | * Whole document * | 1-11, 13-20 | TECHNICAL FIELDS SEARCHED (Int. Cl 4) |
| T | DNA, vol. 8, no. 4, 1989, pages 261-270; M.A. Liebert, Inc. Publ. F. RENTIER-DELRUE et al.: "Tilapia prolactin: molecular cloning of two cDNAs and expression in Escherichia coli" | | |
| | * Whole article. * | | |

The present search report has been drawn up for all claims

| Place of search | Date of completion of the search | Examiner |
|---|---|---|
| | | |